(19) Europäisches Patentamt
European Patent Office
Office européen des brevets

(11) EP 0 645 379 B1

(12) EUROPEAN PATENT SPECIFICATION

(45) Date of publication and mention of the grant of the patent:
**17.03.1999 Bulletin 1999/11**

(21) Application number: **94111896.0**

(22) Date of filing: **29.07.1994**

(51) Int Cl.[6]: **C07D 295/104**, C07D 211/14, C07D 213/50, C07D 209/12, C07D 317/54, C07D 317/64, C07D 295/135, C07C 225/16, C07C 49/255, C07C 311/29, C07C 235/78, A61K 31/445, A61K 31/36, C07F 7/08

(54) **Benzoylacetylene derivatives**

Benzoylacetylen Derivate

Dérivés de benzoylacétylène

(84) Designated Contracting States:
**AT BE CH DE DK ES FR GB GR IE IT LI LU NL PT SE**

(30) Priority: **02.08.1993 JP 191324/93**

(43) Date of publication of application:
**29.03.1995 Bulletin 1995/13**

(73) Proprietor: **Mitsubishi Chemical Corporation**
**Chiyoda-ku Tokyo (JP)**

(72) Inventors:
- **Takayanagi, Hisao**
  **Machida-shi, Tokyo (JP)**
- **Kitano, Yasunori**
  **Yokohama-shi, Kanagawa-ken (JP)**
- **Inokawa, Haruki**
  **Yokohama-shi, Kanagawa-ken (JP)**
- **Suzuki, Tsuyoshi**
  **Yokohama-shi, Kanagawa-ken (JP)**

(74) Representative:
**ter Meer, Nicolaus, Dipl.-Chem., Dr.**
**TER MEER STEINMEISTER & PARTNER GbR,**
**Patentanwälte,**
**Mauerkircherstrasse 45**
**81679 München (DE)**

(56) References cited:

| | |
|---|---|
| **EP-A- 0 169 400** | **EP-A- 0 282 898** |
| **EP-A- 0 476 658** | **DE-A- 2 054 064** |
| **DE-A- 2 060 576** | **GB-A- 2 050 168** |
| **US-A- 3 592 922** | **US-A- 4 708 966** |

- **EUROPEAN JOURNAL OF MEDICINAL CHEMISTRY vol. 14, no. 4 , July 1979 pages 309 - 315 E SCHRÖDER ET AL 'Nichtsteroidale Entzündungshemmer. 3.'**
- **HELVETICA CHIMICA ACTA vol. 69, no. 3 , 1986 pages 560 - 579 M KOLLER ET AL 'Die Gasphasen-Flussthermolyse von 1-Isobutenyl- und 2-Methylphenyl-alkinyl-ketonen'**
- **JOURNAL F. PRAKT. CHEMIE vol. 316, no. 3 , 1974 , LEIPZIG pages 474 - 484 G.W.FISCHER 'Über die Umsetzung von 2-Chlorovinylketonen mit 2-Aminopyridin'**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- TETRAHEDRON vol. 43, no. 1 , 1987 , UK pages 143 - 148 M.ALVARO ET AL 'New photochemical approaches to the synthesis of chromones'
- TETRAHEDRON LETTERS vol. 29, no. 9 , 1988 pages 1045 - 1048 DAWEI MA ET AL 'A novel stereoselective synthesis of conjugated dienones'
- TETRAHEDRON LETTERS vol. 31, no. 16 , 1990 pages 2299 - 2300 D.PFLIEGER ET AL 'Base catalysed cyclisation of 1-(2'-hydroxy-phenyl)-2-ynones'
- TETRAHEDRON LETTERS vol. 27, no. 40 , 1986 pages 4893 - 4894 H.SHENG ET AL 'A novel palladium catalysed reaarangement of acetylenic ketones to furans'
- JOURNAL OF PRGANIC CHEMISTRY vol. 56, no. 18 , 1991 pages 5459 - 5462 J.C.BRADLEY ET AL 'Synthesis of 2-benzylidenebenzocyclobutenones via an intramolecular Stille coupling reaction'
- JOURNAL OF ORGANIC CHEMISTRY vol. 51, no. 23 , 1986 pages 4432 - 4436 H.GARCIA ET AL '5-Endo-dig vs. 5-Exo-dig ring closure in o-Hydroxyaryl phenethynyl ketones'
- JOURNAL OF FORGANIC CHEMISTRY vol. 55, no. 14 , 1990 pages 4349 - 4356 L.MCGARRY ET AL 'Synthesis of some highly functionalised flavones and chromones using cycloacylation reactions and C-3-functionalisation'
- JOURNAL OF ORGANOMETALLIC CHEMISTRY vol. 37, no. 1 , 1 April 1972 pages 45 - 56 D.R.M.WALTON ET AL 'friedel-Crafts reactions of bis(trimethylsilyl)polyynes with acyl chlorides'
- JOURNAL OF THE AMERICAN CHEMICAL SOCIETY vol. 107, no. 2 , 23 January 1985 pages 521 - 522 V.BHASKAR RAO ET AL 'Novel cyclisation of Type II biradicals from alpha,beta-acetylenic ketones'
- JOURNAL OF ORGANIC CHEMISTRY vol. 56, no. 18 , 1991 pages 5459 - 5462 J.C.BRADLEY ET AL 'Synthesis of 2-benzylidenebenzocyclobutenones via an intramolecular Stille coupling reaction'

## Description

### FIELD OF THE INVENTION

This invention relates to benzoylacetylene derivatives and salts thereof, more particularly to benzoylacetylene derivatives and salts thereof having specific protein kinase (referred to as "tyrosine kinase" hereinafter) inhibiting activity.

### BACKGROUND OF THE INVENTION

In chemotherapy for cancer, a lot of therapeutic agents have been put into practical use. However, most of them are not sufficiently effective in their therapeutic action. In addition, growth inhibiting action of them is not limited to cancer cells, and therefore, they often cause serious side-effects, which make them unsatisfactory therapeutic agents.

It is well known that tyrosine kinase plays an important role in intercellular signal transduction and cell differentiation or growth. Accordingly, failure of control of tyrosine kinase activity in cells disorders intercellular signal transduction and causes abnormal cell differentiation/growth, which is considered to be directly responsible for the development of various diseases. In particular, it is known that tyrosine kinase is significantly associated with disorderly overgrowth of cancer cells. In fact, overexpression of tyrosine kinase is epidemiologically observed in various carcinomas.

On the basis of this finding, it has been proposed that an agent specifically inhibiting tyrosine kinase activity would be an anti-cancer agent having minor side-effects and exerting its therapeutic effect through novel mechanisms. Examples of such agent are Erbstatin, Lavendustin, Herbimycin A, and Genistein which are all derived from microorganisms.

Additional example are synthetic compounds such as benzylidenemalonitrile (Japanese Patent Publication Kokai No. 138238/1990; Journal of Medical Chemistry, 32 p2344, 1989; ibid 34 p1896, 1991), α-cyanosuccinamide derivative (Japanese Patent Publication Kokai No. 222153/1988) 3,5-diisopropyl-4-hydroxystyrene derivative (Japanese Patent Publication Kokai No. 39522/1987), 3-5-di-t-butyl-4-hydroxystyrene derivative (Japanese Patent Publication Kokai No. 39523/1987), and Erbstatin analogue (Japanese Patent Publication Kokai No. 277347/1987).

Tyrosine kinase inhibitors previously known possess too moderate inhibitory activity to be used as an anticancer agent. Accordingly, the purpose of the present invention is to provide a family of novel compounds useful for suppressing the growth of cancer cells, which compounds are easily available, exhibit specific and intensive activity in inhibiting tyrosine kinase of the growth factor receptor, and show negligible side-effects that anti-cancer agents previously known have.

### SUMMARY OF THE INVENTION

It has found that a new class of benzoylacetylene derivatives have an unprecedented intensive activity to inhibit tyrosine kinase and have an antiproliferative activity.

According to one aspect of the present invention, there is provided a compound of the following formula (I)

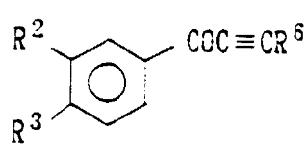

··· ( I )

wherein $R^2$ and $R^3$ are $C_1$-$C_3$ alkoxy; $R^6$ is phenyl, pyridyl or $-CR^{15}R^{16}X$ wherein $R^{15}$ and $R^{16}$ are independently hydrogen or $C_1$-$C_5$ alkyl, optionally substituted by phenyl, or when taken together, they are $C_3$-$C_7$ alkylene, X is hydroxyl or $-NR^{23}R^{24}$ wherein $R^{23}$ and $R^{24}$ are independently hydrogen, phenyl optionally substituted by halogen or $C_1$-$C_5$ alkyl, $C_1$-$C_5$ alkyl optionally substituted by hydroxyl, phenyl or indolyl, pyridyl, $-SO_2R^{27}$ wherein $R^{27}$ is 3,4-dimethoxyphenyl, or $-COR^{25}$ wherein $R^{25}$ is $C_1$-$C_5$ alkoxy optionally substituted by phenyl, pyridyl, N-methylpydridyl or N-oxopyridyl, or when $R^{23}$ and $R^{24}$ are taken together, they are $C_3$-$C_6$ alkylene optionally substituted by $C_1$-$C_5$ alkyl; or its salt, except 2-propyn-1-one, 1-(3,4-dimethoxyphenyl)-3-phenyl.

DETAILED EXPLANATION OF THE INVENTION

The compounds of the present invention are useful as pharmaceutical compositions for suppressing the growth of cancer cells, therapeutical treatment of arterial sclerosis, inhibiting platelet aggregation, immunodepression and suppressing inflammation. They are shown by the above formula (I).

In the general formula (I), halogen includes fluorine, chlorine, bromine and iodine. $C_1$~ $C_5$ alkyl includes methyl, ethyl, n-propyl, iso-propyl, n-butyl, iso-butyl, sec-butyl, tert-butyl, n-pentyl and neopentyl, $C_1$~ $C_5$ alkoxy includes methoxy, ethoxy, n-propoxy, iso-propoxy, n-butoxy, iso-butoxy, sec-butoxy, tert-butoxy, n-pentyloxy and neopentyloxy.

Further, as salts formable from the benzoylacetylene derivatives represented by the above-noted general formula (I), there may be mentioned for example salts of inorganic acids such as carbonates, hydrogencarbonates, hydrochlorides, sulfates and phosphates, or salts of organic acids such as formates, propionates, oxalates, fumarates, maleates, succinates, tartrates, benzoates, phthalates, methansulfonates and 4-toluenesulfonates.

When the benzoylacetylene derivatives have an amino or pyridyl group, oxygen or an alkyl group may combine with nitrogen to form an ammonium salt or pyridinium salt.

Preferred examples of the compounds according to the invention are shown in Table-1. $R^2$ , $R^3$ and $R^6$ in the above-noted general formula (I) are represented in the Table. Ac, Bu, Bn, Me, Et, Pr and Ph represent acetyl group, butyl group, benzyl group, methyl group, ethyl group, propyl group and phenyl group, respectively, in the Table (the same as in the following Tables).

Table - 1

| Compound No. | $R^2$ | $R^3$ | $R^6$ |
|---|---|---|---|
| 1 | OMe | OMe | 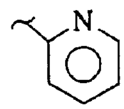 |
| 2 | OMe | OMe | 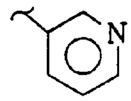 |
| 3 | OMe | OMe | 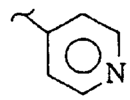 |
| 4 | OMe | OMe | 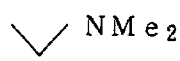 |
| 5 | OMe | OMe | 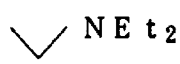 |

Table - 1 (continued)

| Compound No. | $R^2$ | $R^3$ | $R^6$ |
|---|---|---|---|
| 6 | OMe | OMe | 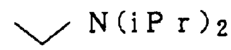 |
| 7 | OMe | OMe | 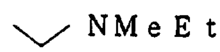 |
| 8 | OMe | OMe | 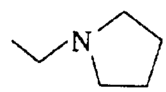 |
| 9 | OMe | OMe | 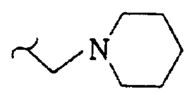 |
| 10 | OMe | OMe | 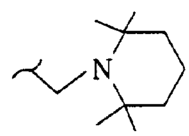 |
| 11 | OMe | OMe | 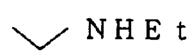 |
| 12 | OMe | OMe | 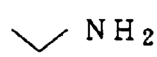 |
| 13 | OMe | OMe | 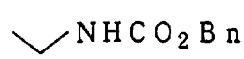 |

Table - 1 (continued)

| Compound No. | $R^2$ | $R^3$ | $R^6$ |
|---|---|---|---|
| 14 | OMe | OMe | 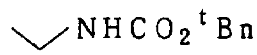 |
| 15 | OMe | OMe | 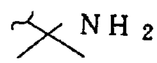 |
| 16 | OMe | OMe | 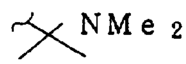 |
| 17 | OMe | OMe | 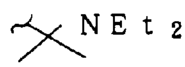 |
| 18 | OMe | OMe | 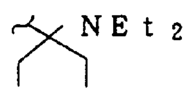 |
| 19 | OMe | OMe | 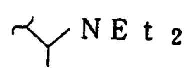 |
| 20 | OMe | OMe | 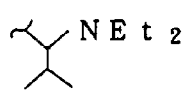 |
| 21 | OMe | OMe | 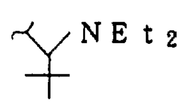 |

Table - 1 (continued)

| Compound No. | $R^2$ | $R^3$ | $R^6$ |
|---|---|---|---|
| 22 | OMe | OMe | 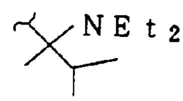 |
| 23 | OMe | OMe | 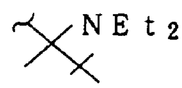 |
| 24 | OMe | OMe | 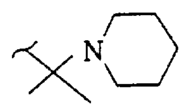 |
| 25 | OMe | OMe | 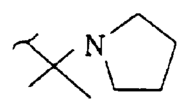 |
| 26 | OMe | OMe | 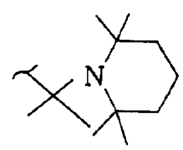 |
| 27 | OMe | OMe | 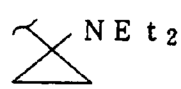 |
| 28 | OMe | OMe | 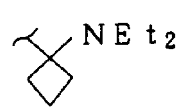 |
| 29 | OMe | OMe | 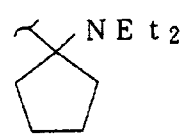 |

Table - 1 (continued)

| Compound No. | $R^2$ | $R^3$ | $R^6$ |
|---|---|---|---|
| 30 | OMe | OMe | 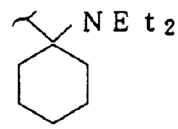 |
| 31 | OMe | OMe | 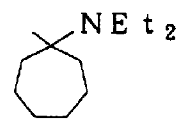 |
| 32 | OMe | OMe | 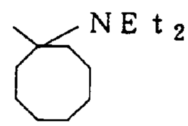 |
| 33 | OMe | OMe | 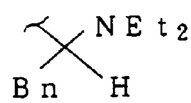 |
| 34 | OMe | OMe | 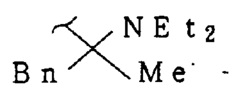 |
| 35 | OMe | OMe | 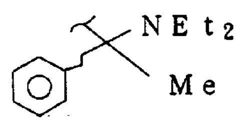 |
| 36 | OMe | OMe | 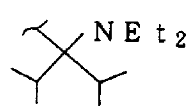 |
| 37 | OMe | OMe | 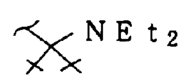 |

Table - 1 (continued)

| Compound No. | $R^2$ | $R^3$ | $R^6$ |
|---|---|---|---|
| 38 | OMe | OMe | 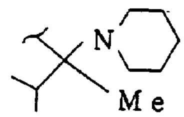 |
| 39 | OMe | OMe | 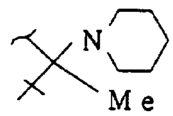 |
| 40 | OMe | OMe | 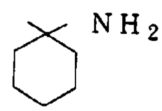 |
| 41 | OMe | OMe | 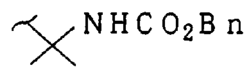 |
| 42 | OMe | OMe | 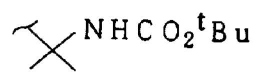 |
| 43 | OMe | OMe | 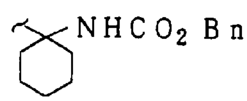 |
| 44 | OMe | OMe | 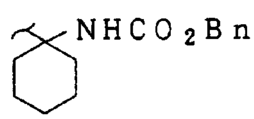 |
| 45 | OMe | OMe | 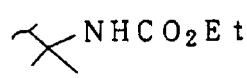 |

Table - 1 (continued)

| Compound No. | $R^2$ | $R^3$ | $R^6$ |
|---|---|---|---|
| 46 | OMe | OMe | 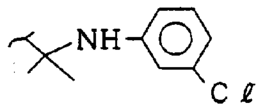 |
| 47 | OMe | OMe | 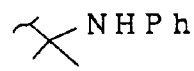 |
| 48 | OMe | OMe | 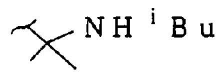 |
| 49 | OMe | OMe | 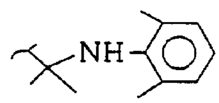 |
| 50 | OMe | OMe | 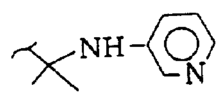 |
| 51 | OMe | OMe | 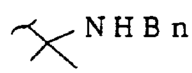 |
| 52 | OMe | OMe | 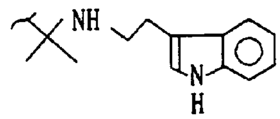 |
| 53 | OMe | OMe | 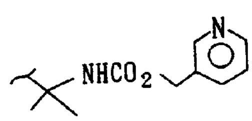 |

Table - 1 (continued)

| Compound No. | $R^2$ | $R^3$ | $R^6$ |
|---|---|---|---|
| 54 | OMe | OMe | 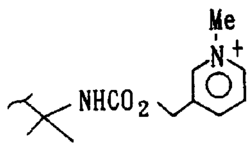 |
| 55 | OMe | OMe | 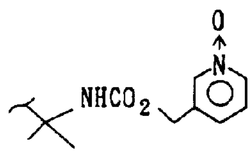 |
| 56 | OMe | OMe | 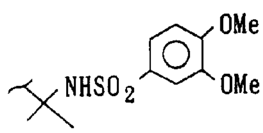 |
| 57 | OMe | OMe | 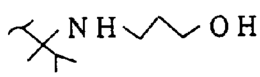 |
| 58 | OMe | OMe | 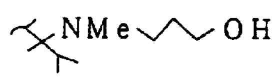 |
| 59 | OMe | OMe | 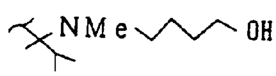 |
| 60 | OMe | OMe | 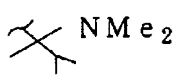 |
| 61 | OMe | OMe | 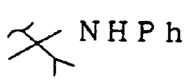 |

Table - 1

| Compound No. | $R^2$ | $R^3$ | $R^6$ |
|---|---|---|---|
| 62 | OMe | OMe | 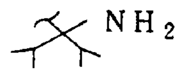 |
| 63 | OMe | OMe | 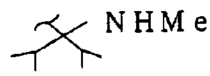 |
| 64 | OMe | OMe | 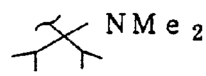 |
| 65 | OMe | OMe | 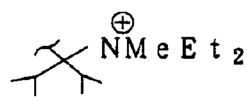 |
| 66 | OMe | OMe | 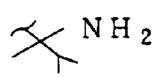 |
| 67 | OMe | OMe | 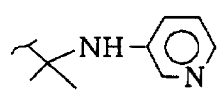 |
| 68 | OMe | OMe | 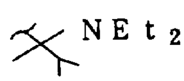 |

Preparation of the compounds of the present invention

The compounds of the present invention can be prepared for example by the following routes:

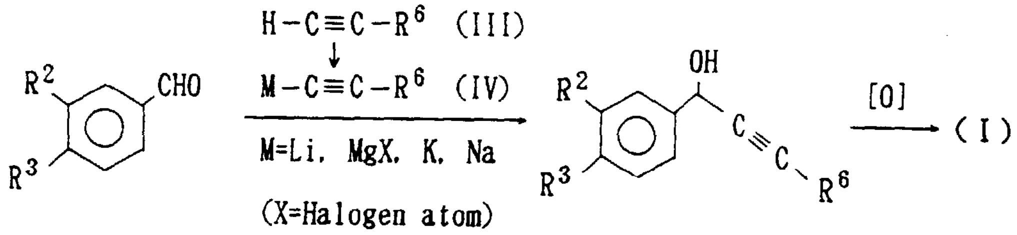


(II) (V)

For example, an acetylene derivative represented by the above-noted general formula (III) can be reacted in a suitable solvent, for example, an ether type solvent such as tetrahydrofuran, diethyl ether, a hydrocarbon type solvent such as benzene, toluene, a proton polar solvent such as methanol, ethanol, as well as a non-proton polar solvent such as dimethyl sulfoxide, and dimethyl formamide, with a base, for example, an organo metal compound such as butyllithium and ethylmagnesium bromide, a metal alcoholate such as sodium methoxide, a metal hydride such as sodium hydride and potassium hydride at a temperature between -100°C and +100°C, preferably -80°C and +50°C, for from 5 minutes to 12 hours to prepare a metal acetylide represented by the general formula (IV), which can be then reacted with a benzaldehyde derivative represented by the general formula (II) at a temperature between -100°C and +100°C, preferably -80°C and +50°C, for from 5 minutes to 24 hours, preferably from 30 minutes to 12 hours, to prepare an adduct represented by general formula (V).

The compounds of the present invention can be prepared by oxidizing the compound (V) in a suitable solvent, for example, a hydrocarbon type solvent such as benzene and toluene, a polar solvent such as acetone and water and a halogen type solvent such as dichloromethane and chloroform. As oxidants used for the oxidation, there may be mentioned metal oxidants such as manganese dioxide and chromic acid, organic oxidants such as oxalyl chloride-trifluoroacetic anhydride. Further, the compound represented by the general formula (I) can be prepared by hydrogen transfer reaction of the compound (V) with aluminium isopropoxide or zirconium chloride in a carbonyl compound. Furthermore, the compounds of the present invention can be prepared by a route shown in the following scheme:

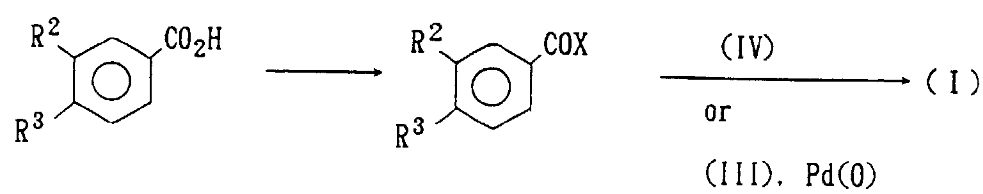


(VI) (VII)

Thus, the compound represented by the general formula (I) can be prepared by reacting a benzoic acid derivative represented by the general formula (VI) with thionyl chloride or phosphorus pentoxide in a hydrocarbon type solvent such as benzene and toluene or a halogen type solvent such as dichloromethane and chloroform to prepare acid halide compounds (VII) and reacting the compounds (VII) with compounds (IV) in an ether type solvent such as tetrahydrofuran and diethyl ether, a hydrocarbon type solvent such as benzene and toluene or a mixed solvent thereof at a temperature between -100°C and +100°C for from 5 minutes to 24 hours.

Furthermorey, the compound represented by the general formula (I) can be prepared by reacting compounds (VI) with compounds (VII) in the presence of a catalytic amount of palladium complex and copper salt in a suitable solvent such as tetrahydrofuran and benzene at a temperature between +10°C and +100°C for from 30 minutes to 48 hours.

Furthermore, the compounds represented by the general formula (I), wherein $R^6$ is $-CR^{15}R^{16}NHCOR^{25}$ ($R^{15}$, $R^{16}$ and $R^{25}$ being as defined above) can be prepared by reacting an acid anhydride or an acid chloride represented by $(R^{25}CO)_2O$ or $ClCOR^{25}$ corresponding to the compound (V) [$R_6 = -CR^{15}R^{16}NH_2$ ($R^{15}$ and $R^{16}$ are defined as above)]

in the presence of bases such as sodium hydroxide, potassium hydroxide, pyridine and triethyl amine in a solvent such as methylene chloride, tetrahydrofuran, toluene and water or a mixed solvent thereof to prepere compounds (V) [-$CR^{15}R^{16}$ $NHCR^{25}$ ($R^{15}$ , $R^{16}$ and $R^{25}$ defined as above)] and then subjecting said compound (V) to the above-noted oxidation reaction.

Furthermore, the compounds represented by the general formula (I), which are a pyridinium salt or an ammonium salt can be prepared in a desired salt form by reacting the corresponding compound having pyridyl group or amide group with the corresponding acid component such as hydrochloric acid, hydrobromic acid or a solution thereof in an organic solvent, fumaric acid or methyl iodide in a suitable solvent such as diethyl ether and chloroform.

Tyrosine kinase inhibitor of the present invention

Tyrosine kinase inhibitor of the present invention comprises at least one compound selected from the compounds represented by the above-noted general formula (I) and their pharmaceutically acceptable salts as an effective ingredient.

The compounds of the present invention are useful as tyrosine kinase inhibitors as described below, and may be used as anti-cancer agent, immunodeppressor, a platelet aggregation inhibitor, an anti-arteriosclerosis or an anti-inflammatory agent based on their activity.

A pharmaceutical composition containing a compound of the formula (I), which is useful as a tyrosine kinase inhibitor can be formulated into various dosage forms for oral, enteral or parenteral administration. Specific formulations include tablets, capsules, fine granules, syrup, suppositories, ointments and injections.

As carriers for preparing such composition in the form of pharmaceutical formulation, there are used pharmaceutical carriers suitable for oral, enteral or parenteral administration, such as organic or inorganic, and solid or liquid substances which are inactive. Specifically, the carriers include, for example, microcrystalline cellulose, gelatin, lactose, starch, magnesium stearate, talc, vegetable fat and oil, animal fat and oil, gum and polyalkylene glycol. The ratio of the compound (I) to a carrier in a formulation can vary in the range of the ratio of 0.2 to 100% by weight.

The pharmaceutical composition of the invention useful as a tyrosine kinase inhibitor may contain other substances effective as a tyrosine kinase inhibitor and other mischible pharmaceuticals. In this case, the compound of the invention may not be a major component of the composition.

The compositions of the invention in the form of a pharmaceutical formulation are generally administered in an amount by which desirable effects can be obtained with no side effect. Particular dosages of the formulation should be determined depending on physician's judgement, but it typically ranges from 10 mg to 10 g, and preferably 20 mg to 5 g, per day for an adult. The compound (I) of the present invention may be administered as an active ingredient in an amount of 1 mg to 5 g, and more preferably 3 mg to 1 g, per day for an adult.

The following detailed examples are presented by way of illustration of certain specific embodiments of the invention. The examples are representative only and should not be construed as limiting the invention in any respect.

[Examples]

Synthesis Example 1

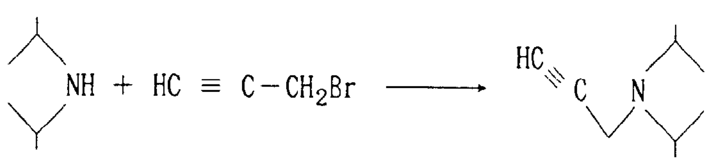

To a suspension of diisopropylamine (4.05g, 40.0mmol) and potassium carbonate (6.91g, 50.0mmol) in acetone (15ml) was added propargyl bromide (3.01ml, 40.0mmol) at 5°C with stirring. The reaction mixture was warmed to room temperature over a period of 2 hours, and then it was stirred at room temperature for further 3 hours. Precipitated substances were filtered off, filtrate was concentrated to give a residue, to which water was added, and a product was extracted with dichloromethane (50ml). After the extract was dried over sodium sulfate, the solvent was distilled off under reduced pressure to yield oily propargyl amine (2.78g, yield 50%). NMR spectra are as follows.

$^1H$ NMR (250MHz, $CDCl_3$) $\delta$ ppm :

1.10 (d, J = 6.5Hz, 12H),

2.13 (t, J = 2.5Hz, 1H),
3.20 (hep, J = 6.5Hz, 2H),
3.42 (d, J = 2.5Hz, 2H).

Synthesis Example 2

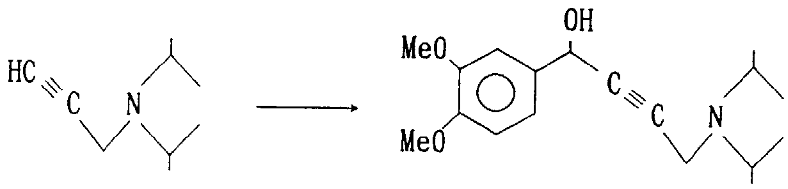

A solution of propargyl amine (1.39g, 10.0mmol) obtained in Synthesis Example 1 in tetrahydrofuran (40ml) was cooled to -70°C under a nitrogen atmosphere, to which a solution of 1.56M n-butyl lithium (6.4ml, 10.0mmol) was added dropwise with stirring. After the reaction solution was warmed slowly to 0°C, it was cooled to -70°C, to which a solution of 3,4-dimethoxy benzaldehyde (1.66g, 10.0mmol) in tetrahydrofuran (10ml) was added dropwise.

After the reaction solution was warmed to 0°C for 2 hours, the reaction was quenched by the addition of water. After the solvent was distilled off under reacted pressure, the residue was extracted with dichloromethane (40ml×2). After the extracts were dried over anhydrous sodium sulfate, the solvent was distilled off under reduced pressure. The residue was purified by silica gel column chromatography (developer: $CHCl_3$/MeOH=20/1) to give the above-noted aminoalcohol (3.00g, yield 98%) as an oily material. NMR spectra are as follows. $^1H$ NMR (250 MHz, $CDCl_3$) δ ppm :

1.10 (d, J = 6.5Hz, 12H),
2.40 (brs, 1H),
3.20 (hep, J = 6.5Hz, 2H),
3.51 (d, J = 1.7Hz, 2H),
3.89 and 3.9 0 (s, 6H),
5.42 (brs, 1H),
6.85 (d, J = 8.1 Hz, 1H),
7.07 (m, 2H).

Synthesis Example 3

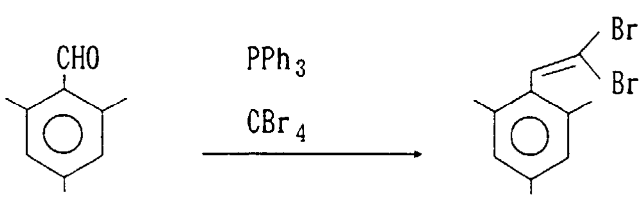


1 2

A solution of triphenyl phosphine (3.54g, 13.5mmol) and carbon tetrabromide (2.24g, 6.7mmol) in methylene chloride (30ml) was cooled to 0t under a nitrogen atmosphere, to which mesitoaldehyde 1 (0.5g, 3.37mmol) was added with stirring. The reaction mixture was stirred at 0°C for 30 minutes and thereafter warmed to the room temperature and stirred at room temperature overnight. Pentane (120ml) was added, the precipitated substances were distilled off, a residue was washed with pentane, a filtrate was concentrated, and the solvent was distilled off. A residue was purified by a silica gel column chromatography (developer: hexane) to obtain 2 (0.310g, yield 30.2%).

Synthesis Example 4

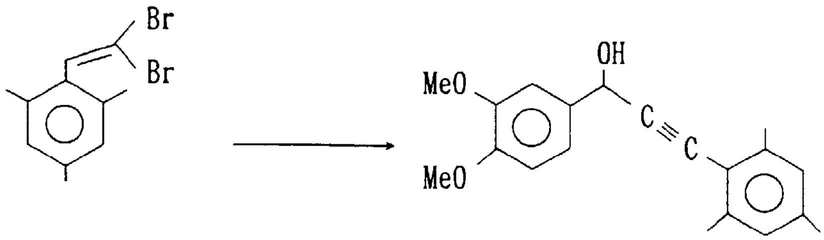

2 3

After a solution of 2 (0.1082g, 0.356mmol) in THF (20ml) was cooled to -78°C under a nitrogen atmosphere, n-butyl lithium (hexane solution, 1.66M, 0.43ml) was added. After the reaction mixture was stirred at -78°C for 1 hour, it was warmed to the room temperature for 1 hour and then stirred at the room temperature for further 1 hour. Thereafter, it was cooled to -78°C again, and veratoraldehyde (0.0592g, 0.356mol) was added and warmed to the room temperature for 2 hours and then stirred at the room temperature for one night. An aqueous ammonium chloride solution was added and the mixture extracted with ethyl acetate. After the extract was dried over sodium sulfate, the solvent was distilled off under reduced pressure. A residue was purified by a silica gel column chromatography (developer: hexane:ethyl acetate=5:1) to give 3 (0.049g, yield 4%).

Synthesis Example 5

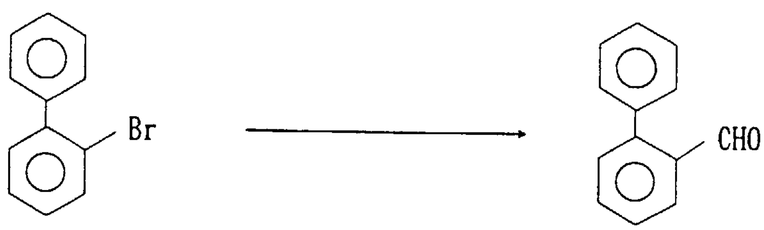

5 6

A solution of 2-bromodiphenyl 5 (1g, 4.29mmol) in THF (40ml) was cooled to -78°C under a nitrogen atmosphere, and n-butyllithium (hexane solution, 1.70M, 3ml) was added with stirring. Alter stirring for 30 minutes, DMF (0.47g, 6.44mmol) was added and warmed to the room temperature for 2 hours and stirred for one night. An aqueous solution of ammonium chloride solution was added and the mixture extracted with ethyl ether. After the extract was dried on sodium sulfate, the solvent was distilled off under a decreased pressure. A residue was purified by a silica gel column chromatography (developer: hexane:ethyl acetate=10:1) to obtain 6 (0.7484g, yield 96%).

Synthesis Example 6

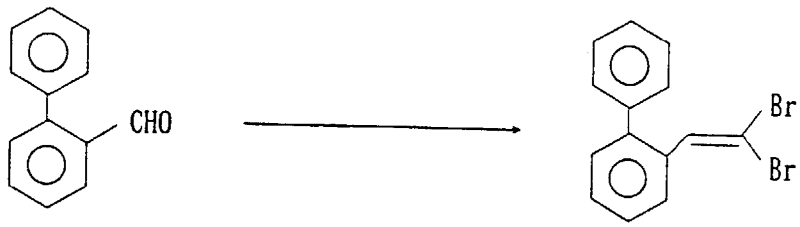

6 7

A solution of triphenyl phosphine (4.3g, 16.4mmol) and carbon tetrabromide (2.72g, 8.2mmol) in methylene chloride (40ml) was cooled to 0°C under a nitrogen atmosphere, and 6 (0.7484g, 4.lmmol) was added with stirring. After the reaction mixture was stirred at 0t for 30 minutes, it was warmed to room temperature and stirred at room temperature over night. To this mixture was added pentane (160ml), and the precipitated substances were filtered off and washed with pentane. The filtrate was concentrated, to give a residue, which was purified by a silica gel column chromatography (developer: n-hexane) to give 7 (1.2058g, yield 87%).

Synthesis Example 7

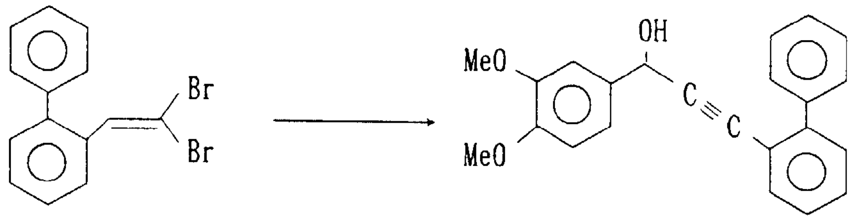

7 8

After a solution of 7 (1.2058g, 3.57mmol) in THF (40ml) was cooled to 0°C under a nitrogen atmosphere, n-butyllithium (hexane solution, 1.70M, 4.0ml) was added with stirring. After the reaction mixture was stirred at -78°C for 1 hour, it was warmed to the room temperature over a period of 1 hour and stirred at the room temperature for further 1 hour. Thereafter it was cooled again to -78°C, veratoraldehyde (0.622g, 3.75mmol) was added and then warmed to the room temperature for 2 hours, and then stirred at the room temperature overnight. An aqueous ammonium chloride solution was added and the mixture extracted with ethyl acetate. After the extract was dried over sodium sulfate, the solvent was distilled off. A residue was purified by a silica gel column chromatography (developer: n-hexane:ethyl acetate=5:1) to give 8 (0.99g, yield 81%).

Example 1

Synthesis of compound No. 6 in Table-1

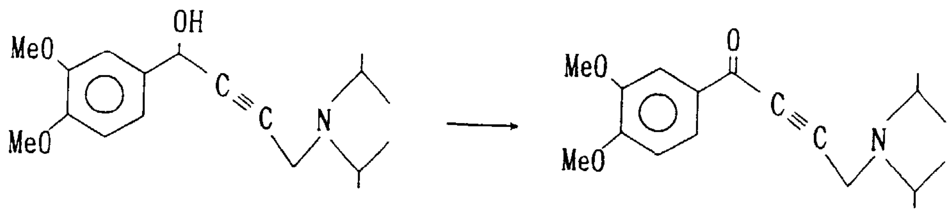

A solution of aminoalcohol (1.20g, 3.93mmol) obtained in Synthesis Example 2 in dichloromethane (50ml) was stirred vigorously at the room temperature, and active manganese dioxide (12.0g) was added to the solution. After 1 hour, insolubles were filtered off, the filtrate was concentrated under reduced pressure, and the residue was purified by neutral alumina column chromatography (developer: n-hexane/ethyl acetate=4/1) to afford the above-noted objective ynone compound (715mg, yield 60 %) as an oil. NMR spectra, form and IR spectra are as follows.

Form: brown oil

$^1$H NMR (250 MHz, $CDCl_3$) δ ppm :

1.16 (d, J = 6.5 Hz, 6H),
3.27 (sep, J = 6.5 Hz, 1H),
3.73 (s, 2H),
3.94 and 3.97 (2s, 6H),
6.94 (d, J = 8.5 Hz, 1H),
7.62 (d, J = 1.8 Hz, 1H),
7.84 (dd, J = 1.8, 8.5 Hz, 1H).

IR (film) $cm^{-1}$ :

2969, 2936, 2209, 1636, 1584, 1514,
1464, 1420, 1343, 1271, 1219, 1175,
1134, 1024, 880, 824, 766, 750, 723.

Example 2

Synthesis of compound No. 9 in Table-1

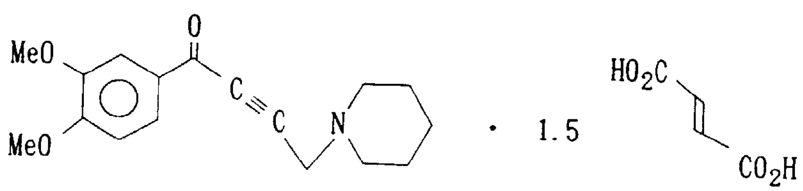

By using piperidine and propargyl bromide as starting materials analogously to Synthesis Examples 1 and 2 as well as Example 1, the corresponding ynone compound was obtained (total yield 7%). A solution of fumaric acid (80mg, 0.69mmol) in acetone (10ml) was added to a solution of the ynone compound (200mg, 0.70mmol) in acetone (5ml) at the room temperature and stirred. After 5 hours, precipitated crystals were filtered off, washed with acetone, and then dried under a decreased pressure to give the above-noted amine fumarate (50mg, yield 18%) as colorless crystals. NMR spectra, melting point and IR spectra are as follows.

mp.: 105-106°C

$^1$H NMR (250MHz, DMSO-d) δ ppm :
1.30-1.70 (m, 6H),
2.51 (m, 4H),
3,67 (s, 2H),
3.84 and 3.88 (2s, 6H),
6. 6 2 (2s, 3 H) ,
7.18 (d, J= 8.5 Hz, 1H),
7.52 (d, J=1.8Hz, 1H),
7.79 (dd, J=1.8, 8.5 Hz, 1H).
IR (film) $cm^{-1}$:
3430, 2938, 2627, 2531, 2236, 1888, 1699, 1638, 1582, 1514, 1456, 1422, 1273, 1219, 1017, 974.

Example 3

Synthesis of compound No. 10 in Table-1

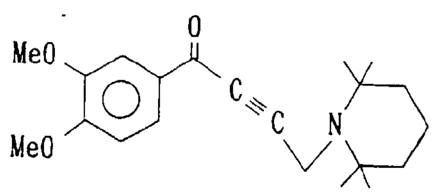

By using 2,2,6,6-tetramethylpiperidine as a starting material analogously to Synthesis Examples 1 and 2 as well as Example 1, the above-noted amine compound was obtained (total yield 18 %). NMR spectra, melting point, form and IR spectra are as follows.
mp.: 142-144°C
Form: light yellow crystals
$^1$H NMR (250 MHz, $CDCl_3$) δ ppm:
1.16 (s, 12H),
1.30-1.65 (m, 6H),
3,60 (s, 2H),
3. 9 5 and 3. 9 7 (2s, 6H),
6.93 (d, J=8.4Hz, 1H),
7.65 (d, J = 2.0Hz, 1H),
7.85 (dd, J = 2.0. 8. 4Hz, 1H).
IR (KBr) $cm^{-1}$ :
2969, 2934, 2868, 2220, 1630, 1582, 1514, 1458, 1345, 1296, 1271, 1219, 1177, 1132, 1019, 858, 766, 617.

Examples 4 ~ 9

By using acetylene compounds (Examples 4 ~ 7) prepared according to the method of A. P. Poisselle et al [J. Org. Chem., 26, 725(1961)] and the method of R. S. Hanze et al [J. Am. Chem. Soc., 82, 4908(1961)] or commercially available acetylene compounds (Examples 8 and 9 ) and veratolaldehyde as starting materials analogously to Synthesis Examples 2 and Example 1, compounds in Example 4 ~ 9 were obtained. Purification in each step was carried out by using a silica gel column chromatography. Structures of acetylene compounds used and objective materials as well as physical property values and yields of objective materials are described as follows. Hydrochloride (Example 5) was obtained by dissolving the ynone compound in ether, adding one equivalent of 4N hydrochloric acid-ethyl acetate solution at an ice-cooled temperature, warming to the room temperature, filtering off and drying under a decreased

pressure.

| Example | Starting material Structural formula | Objective material Structural formula | $^1$H NMR(250MHz, $CDCl_3$) δ ppm | Form etc. | Yield |
|---|---|---|---|---|---|
| 4 | 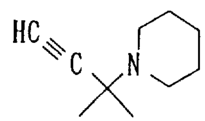 | 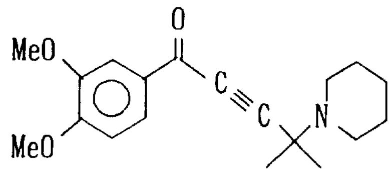 | 1.35-1.50(m, 2H)<br>1.54(s, 6H)<br>1.60-1.80(m, 4H)<br>2.70(brt, J=5.0Hz, 4H)<br>3.95 and 3.98(2s, 6H)<br>6.80(d, J=8.5Hz, 1H)<br>7.65(d, J=1.8Hz, 1H)<br>7.87(dd, J=1.8, 8.5Hz, 1H) | pale yellow oil<br>IR(film)$cm^{-1}$:<br>2978, 2934, 2853<br>2805, 2201, 1638<br>1584, 1514, 1462<br>1418, 1345, 1271<br>1221, 1173, 1132<br>1024, 750 | 75 % |
| 5 | 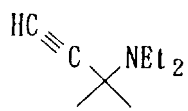 | 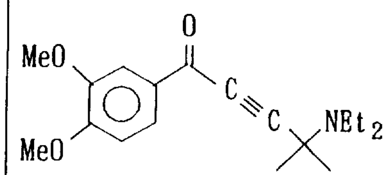<br>· HCℓ | 1.60(t, J=7.4Hz, 3H)<br>2.06(s, 2H)<br>3.29 and 3.55(2m, 2H)<br>3.95 and 3.99(2s, 6H)<br>6.98(d, J=8.5Hz, 1H)<br>7.54(d, J=2.0Hz, 1H)<br>7.81(dd, J=2.0, 8.5Hz, 1H) | colorless powder<br>mp. 178-180°C<br>IR(film)$cm^{-1}$:<br>3445, 2980, 2944<br>2560, 2386, 2224<br>1628, 1580, 1516<br>1472, 1449, 1424<br>1279, 1254 1215<br>1173, 1144 1022 | 72 % |
| 6 | 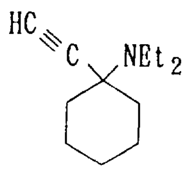 | 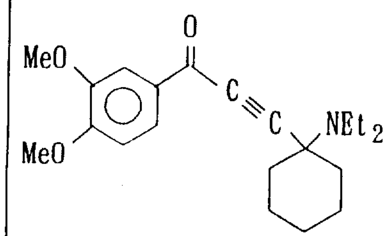 | 1.12(t, J=7.1Hz, 6H)<br>1.50-1.90(m, 8H)<br>2.10-2.25(m, 2H)<br>2.80(q, J=7.1Hz, 4H)<br>3.95 and 3.97(2s, 6H)<br>6.95(d, J=8.5Hz, 1H)<br>7.64(d, J=1.8Hz, 1H)<br>7.85(dd, J=1.8, 8.5Hz, 1H) | pale yellow oil<br>IR(film)$cm^{-1}$:<br>2934, 2859, 2193<br>1636, 1584, 1514<br>1464, 1418, 1271<br>1215, 1175, 1132<br>1024, 750 | 84 % |

| Example | Starting material |  Objective material | | | |
| --- | --- | --- | --- | --- | --- |
| | Structural formula | Structural formula | $^1H$ NMR(250MHz, $CDCl_3$) $\delta$ ppm | Form etc. | Yield |
| 7 | 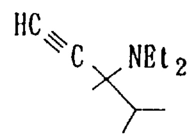 | 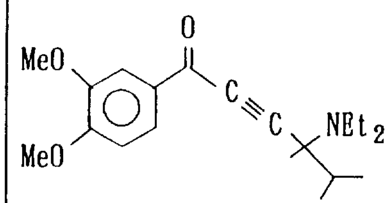 | 0.96(d, J=6.7Hz, 3H)<br>1.09(t, J=7.2Hz, 6H)<br>1.12(d, J=6.7Hz, 3H)<br>2.17(hep, J=6.7Hz, 1H)<br>2.73(m, 4H)<br>3.94 and 3.97(2s, 6H)<br>6.94(d, J=8.4Hz, 1H)<br>7.64(d, J=1.9Hz, 1H)<br>7.84(dd, J=1.9, 8.4Hz, 1H) | pale yellow oil<br>IR(film)$cm^{-1}$:<br>2969, 2197, 1638<br>1584, 1514, 1464<br>1418, 1345, 1271<br>1175, 1132, 1024<br>750 | 12 % |
| 8 | 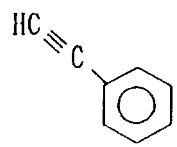 | 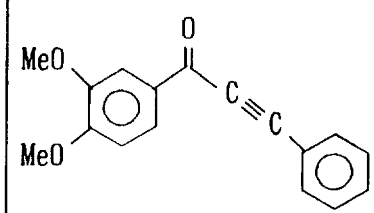 | 3.97 and 3.99(2s, 6H)<br>6.97(d, J=8.4Hz, 1H)<br>7.35-7.50(m, 3H)<br>7.63-7.75(m, 3H)<br>7.96(dd, J=1.9, 8.4Hz, 1H) | pale yellow<br>crystal<br>mp. 92-94°C<br>IR(KBr)$cm^{-1}$:<br>2940, 2209, 1628<br>1584, 1512, 1466<br>1420, 1271, 1163<br>1138, 1017, 893<br>872, 764, 693<br>604, 531 | 24 % |

| Example | Starting material | Objective material | | | |
|---|---|---|---|---|---|
| | Structural formula | Structural formula | $^1H$ NMR(250MHz, $CDCl_3$) δ ppm | Form etc. | Yield |
| 9 | 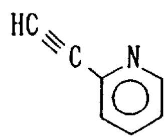 | 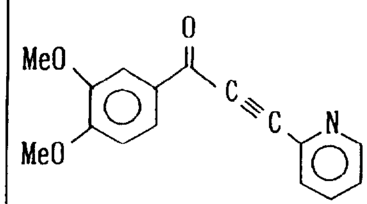 | 3.97 and 3.99(2s, 6H)<br>6.96(d, J=8.4Hz, 1H)<br>7.39(m, 1H)<br>7.65-7.82(m, 2H)<br>8.03(dd, J=2.0, 8.4Hz, 1H)<br>8.72(m, 1H) | yellow solid<br>mp. 102-104℃<br>IR(KBr)$cm^{-1}$:<br>2930, 2216, 1632<br>1595, 1580, 1517<br>1462, 1418, 1302<br>1277, 1252, 1171<br>1136, 1019, 870<br>777, 767, 747<br>720, 610 | 66 % |

Reference Example 1

Synthesis of compound No. 197

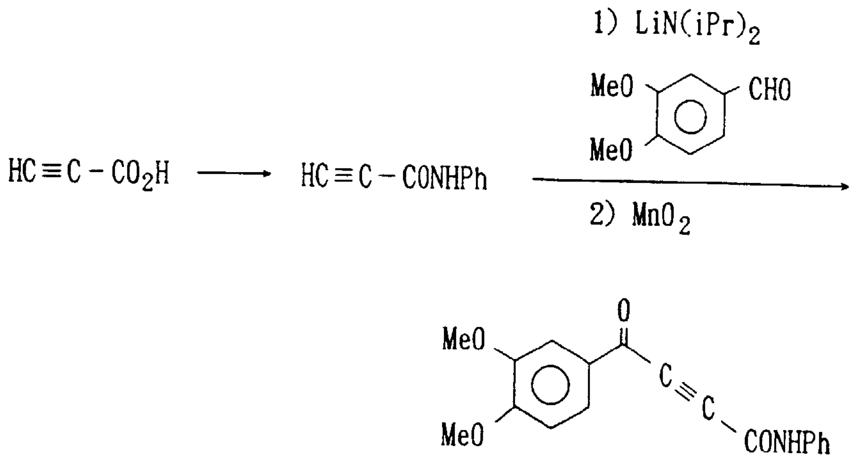


(197)

A solution of propiolic acid (5.64g, 80mmol) in tetrahydrofuran (100ml) was stirred on an ice-bath, triethylamine (13.4ml, 96mmol), aniline (8.0ml, 88mmol) and phosphorus oxychloride (8.2ml, 88mmol) were added. The reaction mixture was stirred for one night at room temperature, to which water was added with ice cooling. The solvent was distilled off under reduced pressure, and the mixture was extracted with ethyl acetate (50ml×2). The extracts were concentrated and thereafter the residue was purified by a silica gel column chromatography (developer: chloroform/ethyl acetate=2/1) to yield an anilide compound (5.58g, yield 48%).
A solution of the anilide compound (492mg, 3.37mmol) in tetrahydrofuran (20ml) was cooled to -70°C, and 2M a solution of litium diisopropyl amide in cyclohexane (3.54ml, 7.08mmol) was added slowly dropwise. After stirring for 1 hour, 3,4-dimethoxy benzaldehyde (560mg, 3.37mmol) was added.
The resulting solution was warmed to room temperature for 1 hour, then cooled to 0°C, and thereafter water (10ml) was added. After the solvent was distilled off under reduced pressure, water (30ml) was added to the residue and the product was extracted with ethyl acetate (50ml). After the extract was dried (anhydrous sodium sulfate) and concentrated, the residue obtained was purified by a silica gel column chromatography (developer: chloroform/ethyl acetate=4/1) to obtain an adduct, an alcohol compound (600mg, 57%).
The alcohol compound (580mg, 1.86mmol) was subjected to oxidation reaction analogously to Example 1, and the crude product obtained was purified by a silica gel column chromatography (developer: n-hexane/chloroform/ethyl acetate=1/1/1) and washed with ether-hexane and filtered off to give the above-noted objective ketone compound (85 mg, yield 14%). NMR spectra, form, melting point and IR spectra are as follows.
Form: light orange powder
mp.: 140-142°C

$^1$H NMR (250 MHz, $CDCl_3$) δ ppm :
- 3. 93 and 3. 9 7 ( 2 s, 6 H) ,
- 6.75 (d, J=8.5Hz, 1H),
- 7.19 (t, J = 7.6 Hz, 1H),
- 7.37 (m, 2H),
- 7.54 (d, J=1.8Hz, 1H),
- 7.60 (d, J=8.2Hz, 2H),
- 7.83 (dd, J = 1.9, 8.5Hz, 1H),
- 8.36 (brs, 1H).

IR (KBr) cm$^{-1}$ :
3324, 1680, 1630, 1599 1584, 1545,
1510, 1462, 1442, 1420 1321, 1271,
1171, 1144, 1020, 874, 760.

Example 10

Synthesis of compound No. 15 in Table-1

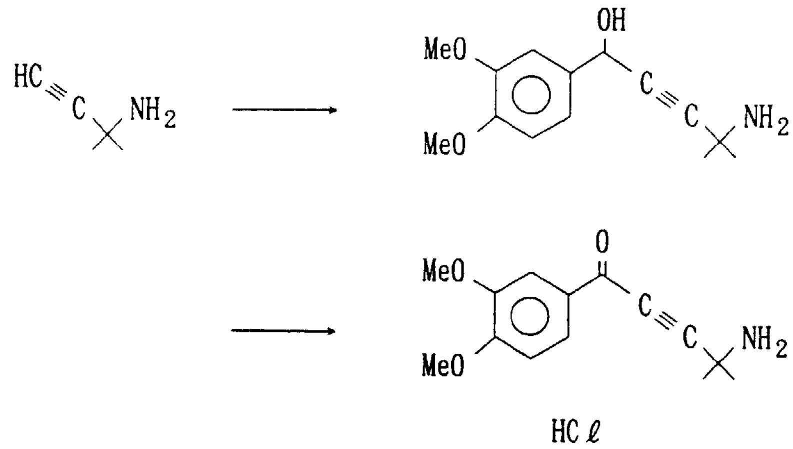

Molecular sieve 4A (10g) was added to a solution of 3-amino-3-methyl-1-butene (5.0g, 90% in $H_2O$, 54mmol) in tetrahydrofuran (50ml) and stirred for 30 minutes under a nitrogen atmosphere. Under a nitrogen atmosphere, molecular sieve was filtered off and the filtrate was cooled with a dry ice-ethanol bath. Then 1. 63M solution of n-butyllithium in hexane (33.0ml, 54.0mmol) was added.

After warming the reaction mixture slowly to 0t, it was cooled again with the dry ice-ethanol bath and water was added to stop reaction. After distilling off the solvent under a decreased pressure, water was added and a product was extracted with dichloromethane (70ml×2). The extracts were dried (anhydrous sodium sulfate) and concentrated, and the obtained oily material was purified by a silica gel column chromatography (developer: chloroform/methanol=50/3) to obtain an adduct, aminoalcohol (7.65g, 57%). NMR spectra, form and melting point of the aminoalcohol are as follows.

$^1H$ NMR ($CDCl_3$, 250MHz) δ ppm:

1.44 (s, 6H),
1.98 (brs, 3H),
3. 8 9 and 3.90 (2s, 6H),
5.41 (s, 1H),
6.86 (d, J = 8.7 Hz, 1H),
7.03 - 7. 1 0 (m, 2H).

Form: light yellow crystal
mp.: 85-87°C

Active manganese dioxide (10.2g) was added in 10 portions to a solution of the above-noted aminoalcohol (1.65g, 6.59mmol) in dichloromethane (50ml) at the room temperature. The reaction mixture was stirred for 2 minutes and filtered by a pad of celite. After concentration of the filtrate, the residue was dissolved in diethyl ether (30ml). The resulting solution was stirred under a nitrogen atmosphere on an icebath, and a 4N hydrochloric acid-ethyl acetate solution (1.8ml) was dropwise added and stirred at the same temperature for 10 minutes.

The precipitated product was filtered off, dried, and suspended in ethyl acetate-diethyl ether (5/1). After stirring for 30 minutes and filtering off, the objective 4-amino-1-(3,4-dimethoxy)phenyl-1-oxo-4-methyl-2-pentyne hydrochloride (970mg, 52%) was obtained. NMR spectra, IR spectra, form and melting point are as follows.

$^1H$ NMR ($CDCl_3$, 250 MHz) δ ppm :

1.91 (s, 6H),
3.89 and 3.91 (2s , 6H),
6.87 (d, J = 8.4 Hz, 1H),
7.42(s, 1H),
7.90 (d, J = 8.4 Hz, 1H).

IR (KBr) $cm^{-1}$ :

3424, 2940, 2843, 2234, 1636, 1584,

1514, 1464, 1422, 1302, 1275, 1217,
1175, 1146, 1019, 748

Form: yellow powder

mp.: 139-141°C

Example 11

Synthesis of compound No. 41 in Table-1

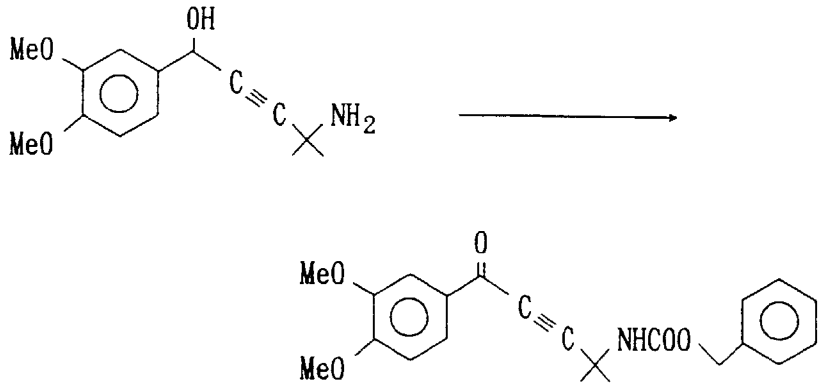

An 10% aqueous sodium carbonate solution (20ml) and dichloromethane (10ml) were added to the intermediate aminoalcohol (738mg, 2.96mmol) synthesized in Example 10, then the mixture was stirred vigorlously with ice cooling, and benzyl chloroformate (0.51ml, 3.55mmol) was added. After the reaction mixture was warmed slowly to the room temperature, a product was extracted with dichloromethane (50ml) and the extract was washed with brine.

The extract was dried (anhydrous sodium sulfate) and concentrated, the residue was dissolved in dichloromethane (60ml), and then active manganese dioxide (6g) was added with stirring at the room temperature. After stirring for 10 min. the mixture was filtered and the filtrate was concentrated. The residue was purified by a silica gel column chromatography (developer: n-hexane/ethyl acetate/chloroform=4/1/1) to give the required carbamate compound (654mg, 58%) as an oil. NMR spectra and IR spectra are as follows.

$^1$H NMR ($CDCl_3$, 250 MHz) δ ppm :

1.72 (s,6 H),
3.94 and 3.95 (2s, 6H),
5.13 (brs, 3H),
6.87 (d, J = 8.5 Hz, 1H),
7.20 - 7.40 (m, 5H),
7.66 (d, J = 1.8Hz, 1H),
7.87 (dd, J = 1.8, 8.5 Hz, 1H).

IR (film) $cm^{-1}$:

3349, 2982, 2940, 2222, 1725, 1634,
1584, 1514, 1454, 1420, 1345, 1271,
1132, 1076, 1022, 876, 750.

Example 12

Synthesis of compound No. 64 in Table-1

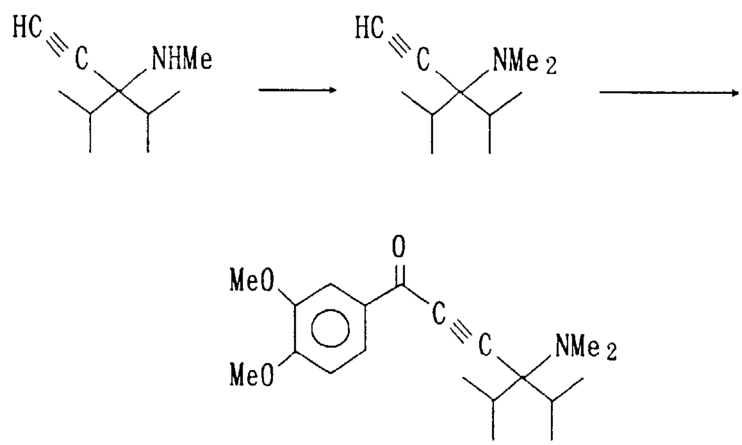

An acetylene compound synthesized according to the method by A. P. Poisselle et al [J. Org. Chem., 26, 725 (1961)] and the method by R. S. Hanzel et al [J. Am. Chem. Soc., 82, 4908 (1961)], [3-(methylamino)-3-isopropyl-4-methyl-1-pentyne] (1.53g, 10.0mmol), formalin (2ml, 37%) and formic acid (0.75g) were refluxed for 1.5 hours. After the reaction solution was cooled, it was made basic with a 2N-NaOH solution and then extracted with ether (30ml×1, 20ml×1). The extracts were dried ($NaSO_4$) and concentrated under reduced pressure. The residue (920mg) was reacted with veratoraldehyde (850mg, 5.1mmol) analogously to Synthesis Example 2 and Synthesis Example 3 to obtain the above-noted objective inone compound (1.00g, yield 30%) as colorless cubic crystals.
mp. 87~88°C

$^1$H NMR (250MHz, $CDCl_3$) δ ppm :

1.12 and 1.14 (2 d, J = 6.7Hz, 12H),
2.30 (hep, J = 6.7Hz, 2H),
2.53 (s, 6H),
3.94 and 3.97 (2s, 6H),
6.94 (d, J = 8.4 Hz, 1H),
7.67 (d, J = 1.9 Hz, 1H),
7.87 (dd, J = 8.4, 1.9 Hz, 1H),

IR (KBr) $cm^{-1}$:

2963, 2872, 2830, 2785, 2199, 1628,
1595, 1581, 1512, 1466, 1420, 1267,
1177, 1140, 1088, 1020, 895, 818, 766,
750, 615.

Example 13

Synthesis of compound No. 65 in Table-1

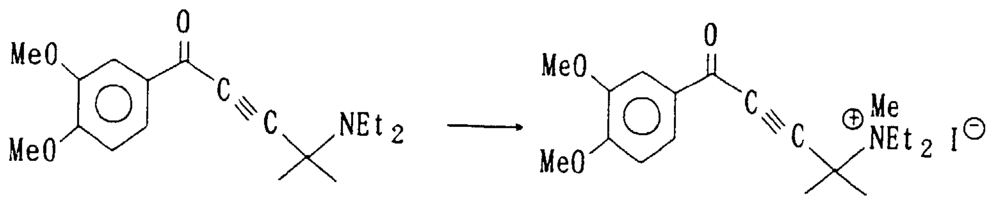

Indomethane (1 ml) was added to a solution of the ynone compound (100mg, 0.33mmol) synthesized in Example 5 in toluene, and stirred at 80°C for 6 hours. The solution was cooled to the room temperature, ether (2ml) was added and heated to reflux for 5 minutes. The mixture was cooled to the room temperature and the above-noted objective compound was collected by filtration.
mp.

$^1$H NMR (300 MHz, $CDCl_3$) δ ppm:
1.61 (t, J = 8.2 Hz, 6H),
2. 1 4 (s, 6H),
3.37 (s, 3H),
3.88 (m, 4H),
3. 9 5 and 4. 0 0 (2s, 6H),
7. 0 5 (d, J = 8.4 Hz, 1H) ,
7. 5 1 (d, J = 2.0 Hz, 1H),
7.72 (dd, J = 8.4, 2.0Hz, 1H).

Example 14

Synthesis of compound No. 53 in Table-1

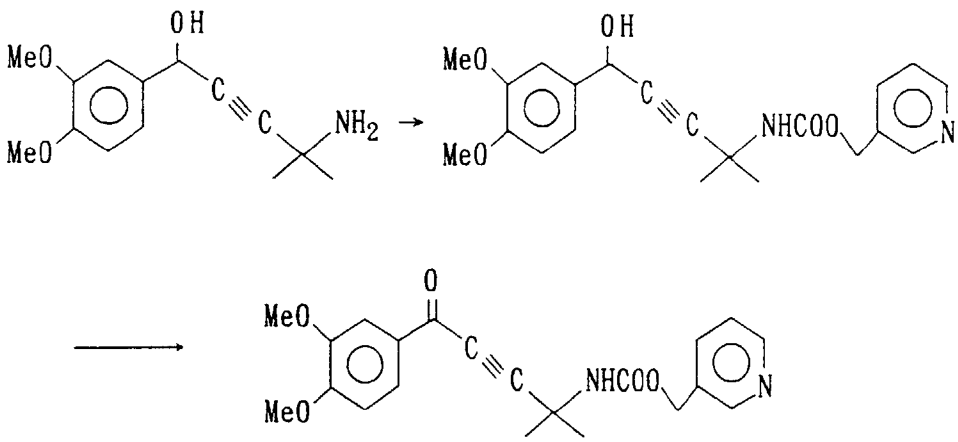

Disuccinyl carbonate (640mg, 2.5mmol) was added to a solution of 3-pyridine methanol (240ml, 2.5mmol) in dichloromethane (10ml) and the mixture stirred for one night at the room temperature. To this mixture was added a solution of aminoalcohol [4-amino-1-(3,4-dimethoxy)phenyl-4-methyl-2-pentyn-1-ol] (500mg, 2.0mmol) in dichloromethane (5ml) and stirred overnight. Water was added to the reaction mixture and the product was extracted with ethyl acetate. The extract was dried ($Na_2SO_4$) and concentrated, and the residue was purified by a silica gel column

chromatography (developer: chloroform/methanol=50/1) to give a pyridylmethyl carbamate compound (650mg, 67%). $^1$H NMR of the pyridylmethyl carbamate compound is as follows.

$^1$H NMR (250MHz, $CDCl_3$) δ ppm :

1.62 (s, 6H),
3.86 (2s, 6H),
5.06 (s, 2H),
5.43 (s, 1H),
5.48 (b r s, 1H),
6.81 (d, J = 8.3 Hz, 1H),
7.07 (d d. J = 8.3, 1.8 Hz, 1H),
7.13 (d, J = 1.8Hz, 1H),
7.23 (dd, J = 4.7, 7.8 Hz, 1H),
7.63 (dt, J = 7.8, 1.9 Hz, 1H),
8.47 (dd, J = 4.7, 1.5Hz, 1H),
8.55 (m, 1H).

The above-noted pyridylmethyl carbamate compound was oxidized analogously to the method of Synthesis Example 3 to obtain an ynone compound (yield 82%) as a brown oil.

$^1$H NMR (250MHz, DMSO-d+1 drop $D_2O$) δ ppm :

1.63 (s, 6H),
3.84 and 3.90 (2s, 6H),
5.16 (s, 2H),
7.09 (d, J = 8.5 Hz, 1H),
7.43 (m, 1H),
7.55 (d, J = 1.6 Hz, 1H),
7.77 (dd, J = 8.5, 1.6 Hz, 1H),
7.83 (d, J = 7.9 Hz , 1H),
8.20 (s, 1H),
8.53 (brd, J = 4.1 Hz),
8.61 (brs, 1H).

IR (film) $cm^{-1}$ :

3345, 3216, 2984, 2220, 1725, 1634,
1583, 1515, 1464, 1422, 1345, 1271,
1082, 1022, 876, 752.

Example 15

Synthesis of compound No. 55 in Table-1

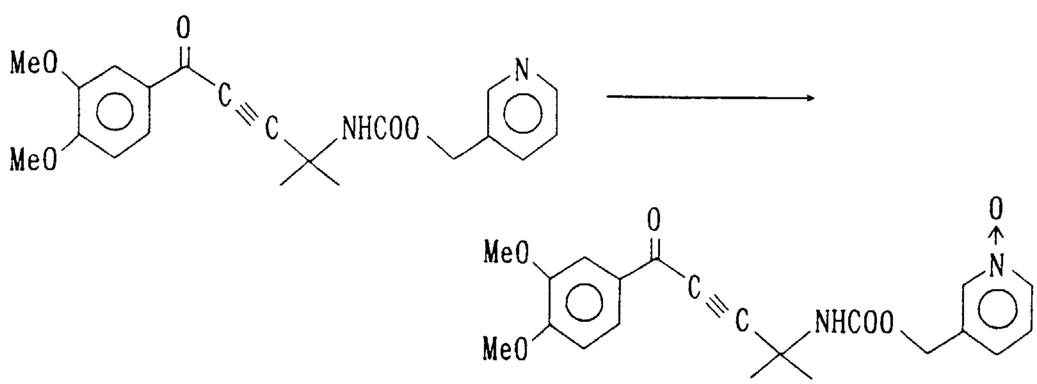

Magnesium monoperoxyphthalate (240mg) was added to a solution of the ynone compound (189mg, 0.49mmol) in dichloromethane (5ml) with ice cooling and stirred for 2 hours at the room temperature. Again, magnesium monoperoxyphthalate (240mg) and ethanol (5ml) were added with ice cooling and stirred for 2 days at room temperature. Water was added to the reaction mixture and a product was extracted with dichloromethane (15ml×2), and the

extract was washed with a saturated aqueous sodium hydrogensulfite solution and dried ($Na_2SO_4$). The solvent was distilled off under reduced pressure to give the objective N-oxide compound (178mg, 92%) as light yellow crystals.
mp. 65~80°C (not show clear melting point)

$^1$H NMR (250 MHz, DMSO-d+1 drop $D_2O$) δ ppm :

1.63 (s, 6H),
3.84 and 3.89 (2s,6H),
5.10 (s, 2H),
7.11 (d, J=8.5Hz, 1H),
7.45 (m, 2H),
7.54 (d, J=1.6 Hz, 1H),
7.77 (dd, J=1.6, 8.5Hz, 1H),
8.20 (m, 1H),
8.31 (brs, 1H).

IR (KBr) $cm^{-1}$ :

3347, 2982, 2940, 2361, 2220, 1724,
1636, 1584, 1514, 1271, 1171, 1086,
1020, 752.

Example 16

Synthesis of compound No. 54 in Table-1

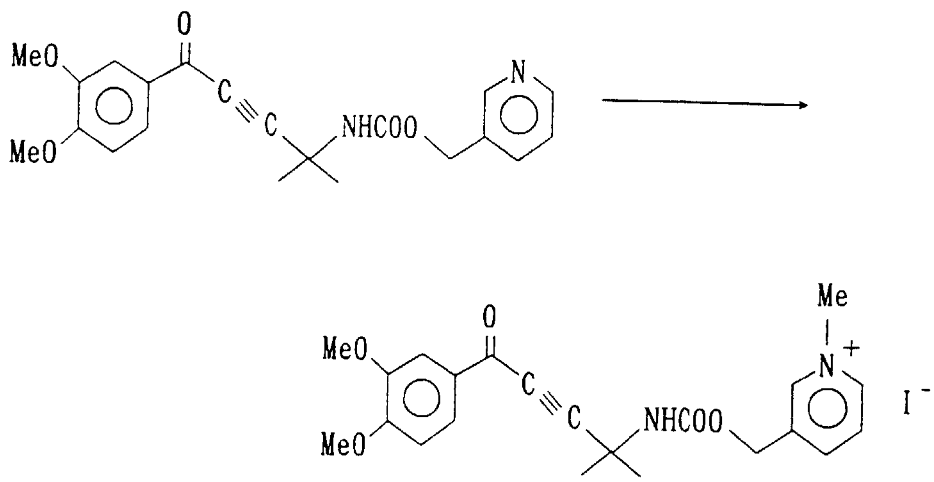

Methyl iodide (0.5ml) was added to a solution of the inone compound (100mg, 0.26mmol) synthesized in Example 14 in ether (5ml) and stirred for 3 days. The precipitates were collected by filtration and dried under reduced pressure to give the objective pyridinium compound (70mg, 51%) as light yellow powdery crystals.
mp. 196~197.5°C

$^1$H NMR (250 MHz, DMSO - d + 1 drop $D_2O$) δ ppm :

1.64 (s,6H),
3.84 and 3.88 (2s, 6H),
4.34 (s,3H),
5.20 and 5.35 (2d, J = 36.0 Hz, 2H),
7.13 (d, J=8.5Hz, 1H),
7.54 (s,1H),
7.78 (s, J=8.2 Hz, 1H),
8.11 (m,1H),
8.55 (d,J=8.0Hz, 1H),
8.90 (d, J=6.0 Hz, 1H),
9.00 (s, 1H).

Example 17

Synthesis of compound No. 14 in Table-1

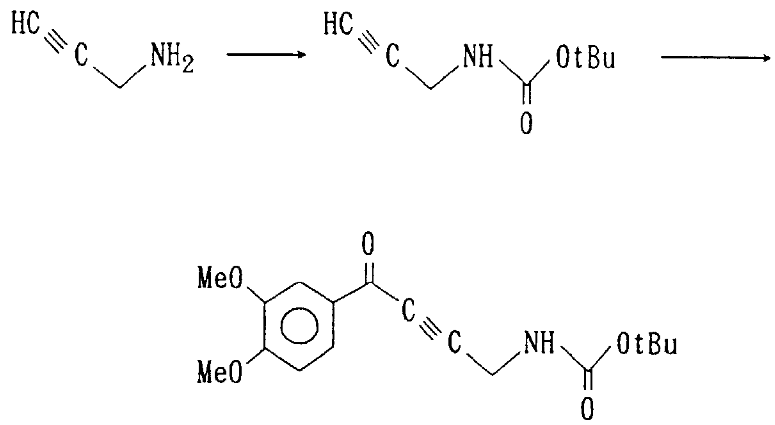

A solution of propargylamine (1.65g, 30mmol) in 1N NaOH was stirred on an ice-bath, and di-t-butyl carbonate (6.9ml, 30.0mmol) was added. It was warmed to the room temperature and stirred overnight. It was extracted with ether, and the extract was washed and dried ($Na_2SO_4$). The solvent was distilled off under a decreased pressure to give a carbamate compound (3.57g, 77%). The obtained carbamate compound was oxidized by the method analogously to Reference Example 1, and reacted with veratrol, and thereafter purified by a silica gel column chromatography (developer: n-hexane/ethyl acetate=1/1) to afford the objective ynone compound (yield 45%) as light yellow crystals.
mp. 110~113°C

$^1$H NMR (250MHz, $CDCl_3$) δ ppm :

1.48 (s, 9H),
3.94 and 3.97 (2s, 6H),
4.22 (d, J=5.6 Hz, 2H),
4.94 (brs, 1H),
6.91 (d, J = 8.5 Hz, 1H),
7.59 (d, J=1.8 Hz, 1H),
7.83 (dd, J=1.8, 8.5 Hz, 1H).

IR (KBr) $cm^{-1}$ :

3351, 3007, 2940, 2836, 2234, 1682,
1620, 1597, 1581, 1508, 1454, 1416,
1354, 1267, 1028, 866, 833, 766.

Example 18

Synthesis of compound No. 45 in Table-1

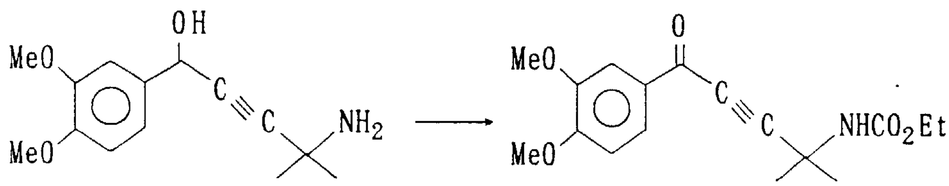

Aminoalcohol (3.34g, 13.4mmol) synthesized in Example 10 was reacted with ethyl chloroformate (1.54ml, 16.1mmol) by the method analogously to Example 11, and thereafter oxidized with manganese dioxide (12.2g). The crude product was purified by a silica gel column chromatography (developer: n-hexane/ethyl acetate=2/1) to give the objective title compound (4.27g, quantative) as colorless cubic crystals.
mp. 72.0~73.5°C

$^1$H NMR (250 MHz, $CDCl_3$) δ ppm:
1.26 (t, J=7.1 Hz, 3H),
1.72 (s, 6H),
3.97 (s, 6H),
4. 1 4 (q, J=7.1 Hz, 2H),
4. 9 6 (brs, 1H),
6.93 (d, J = 8.5Hz, 1H),
7.68 (d, J = 1.9 Hz, 1H),
7. 9 0 (dd, J = 8.5, 1.9 Hz, 1H).

IR (KBr) $cm^{-1}$ :
3329, 2982, 2228, 1709, 1626, 1591, 1512, 1464, 1421, 1211, 1173, 1146, 1084, 1020, 756.

Example 19

Synthesis of compound No. 56 in Table-1

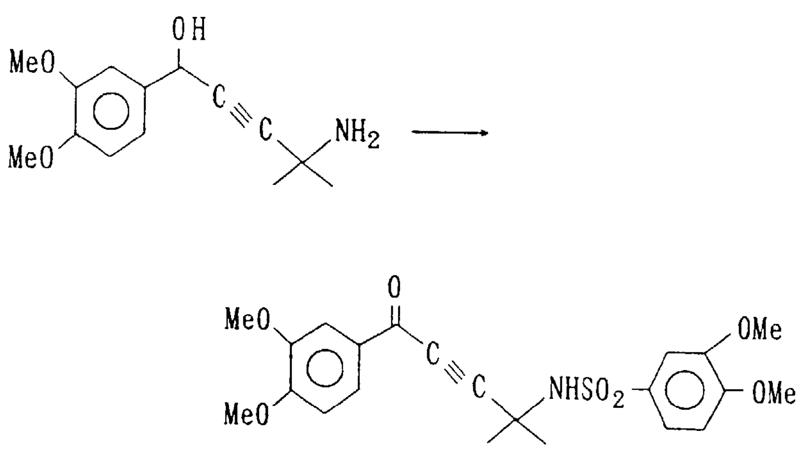

By using aminoalcohol synthesized in Example 10 and 3,4-dimethoxy sulfonyl chloride in the method analogously to Example 11, sulfonamidation and oxidation were carried out to convert to an ynone compound. The crude product was purified by a silica gel column chromatography (developer: chloroform/methanol=75/1) to give the titled compound as a brown oil (yield 13%).

$^1$H NMR (250 MHz, $CDCl_3$) δ ppm :
1.67 (s, 6H),
3.80 and 3.84 (2s, 6H),
3.95 and 3.97 (2s, 6H),
5.06 (brs, 1H),
6.79 (d, J=8.6 Hz, 1H),
6.91 (d, J=8.4 Hz, 1H),
7.36 (d, J=2.2 Hz, 1H),
7.54 (dd, J=2.2 Hz, 8.6 Hz, 1H),
7.57 (d, J=1.9 Hz, 1H),
7.68 (dd, J=1.9, 8.4Hz, 1H).

IR (film) $cm^{-1}$:
3266, 3021, 2940, 2841, 2220, 1636, 1589, 1512, 1464, 1271, 1140, 1022, 754.

Example 20

Synthesis of hydrochloride free compound No. 5 in Table-1

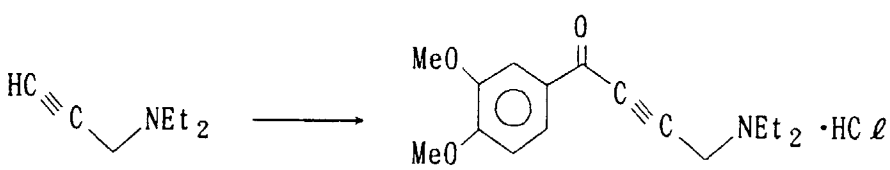

The starting material, 3-diethylamino-1-propyne, was prepared according to Synthesis Example 1, from which the above-noted ynone compound (free hydrochloride) was synthesized (yield 47%). 3.0g (10.89mmol) of the above-noted ynone compound (free hydrochloride) was dissolved in 200ml of methylene chloride, and 5.8ml of a solut of 4N-hydrochloric acid-ethyl acetate was added dropwise under a nitrogen atmosphere at 0°C. It was stirred for 20 minutes, then filtered, and dried under reduced pressure to give 2.9g (85%) of the above-noted hydrochloride of the inone compound.

m.p. 140-142°C

$^1$H NMR (250MHz, $CDCl_2$) δ ppm :
1.55 (t, 6H, J=7.3 Hz),
3.08-3.23 (m, 4H),
3.96 (s, 3H),
3.99 (s, 3H),
4.23 (d, 2H, J=2.1 Hz),
6.98 (d. 1H, J=8.4 Hz),
7.55 (d. 1H, J = 2.0Hz),
7.86 (dd, 1H, J=8.3.2.0Hz),
13.22 (brs, 1H).

IR (film) $cm^{-1}$ :
3426, 2946, 2922, 2637, 2556, 2486, 2228, 1638, 1580, 1516, 1470, 1449, 1343, 1298, 1277, 1219, 1138, 1017, 860, 829.

Examples 21-36

By using various acetylene compounds synthesized according to the method by A. P. Poisselle et al [J. Org. Chem., 26, 725(1961)] and the method by R. S. Hanzel et al [J. Am. Chem. Soc., 82, 4908(1960)] and various aldehydes as starting materials in the methods analogously to Synthesis Example 2 and Example 1, various ynone compounds were synthesized. Purification in each step was carried out by using a silica gel column chromatography. Hydrochlorides were obtained by adding one equivalent of a solut of hydrochloric acid-ethyl acetate warming to the room temperature, filtering off and drying under reduced pressure. Starting materials used as well as physical properties and the yield of the objective materials are described as follows.

| Example | Starting material | Objective material | | | | |
|---|---|---|---|---|---|---|
| | Structural formula | Structural formula | $^1$HNMR | IR(cm-1) | mp. Form | Yield(%) |
| 21 | 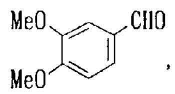 , 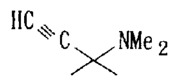 | 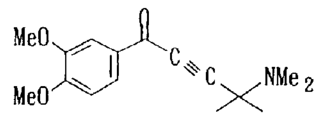 | (DMSO-$d_6$ 250MHz) δ ppm; 1.80(3H, s), 2.85(3H, s), 3.86(3H, s), 3.40(3H, s), 7.16(1H, d, J=8.5Hz), 7.50(1H, d, J=2Hz), 7.89(1H, dd, J=8.5, 2Hz), 12.06(1H, br) | 3429 2924 2845 2640 2550 2461 2363 2227 1626 1595 1581 1514 1467 1423 1394 1354 1294 1278 1246 1217 1176 1140 1070 1018 918 871 815 765 | mp. 171～173 ℃ white crystal | 37 |
| 22 | 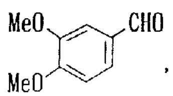 , 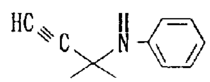 | 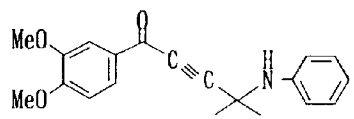 · HCℓ | (DMSO-$d_6$ 250MHz) δ ppm; 1.75(6H, s), 3.75(3H, s), 3.88(3H, s), 4.05 ～4.95(2H, br), 6.99 ～7.11(1H, m), 7.09(1H, d, J=8.5Hz), 7.19 ～7.37(4H, m), 7.43(1H, d, J=1.8Hz), 7.70(1H, dd, J=8.5, 1.8Hz) | 3431 2937 2843 2781 2650 2540 2444 2224 1628 1595 1581 1516 1466 1421 1277 1249 1211 1143 1070 1018 889 866 842 819 781 765 746 734 | mp. 139～140 ℃ white crystal | 74 |

| Example | Starting material | | Objective material | | | |
|---|---|---|---|---|---|---|
| | Structural formula | Structural formula | $^1$HNMR | IR(cm-1) | mp. Form | Yield(%) |
| 23 | 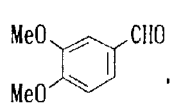 , 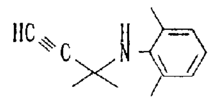 | 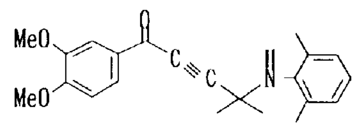 | (CDCl$_3$ 250MHz) δ ppm; 1. 65(6H, s), 2. 41(6H, s), 3. 15(1H, bs), 3. 89(3H, s), 3. 96(3H, s), 6. 79(1H, d, J=8. 5Hz), 6. 98(1H, dd, J=5. 9, 5. 9Hz), 7. 06(1H, d, J=5. 9Hz), 7. 07(1H, d, J=5. 9Hz), 7. 37(1H, dd, J=8. 5, 2. 0Hz), 7. 51(1H, d, J=2. 0Hz) | 3350 3001 2937 2361 2208 1880 1624 1579 1512 1466 1452 1421 1277 1249 1209 1170 1141 1068 1022 972 914 868 823 790 763 746 711 638 | mp. 100~101 ℃ white crystal | 63 |
| 24 | 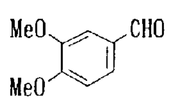 , 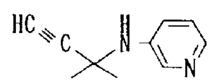 | 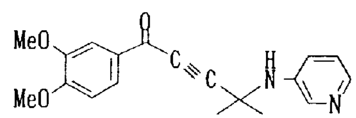 | (CDCl$_3$ 250MHz) δ ppm; 1. 76(6H, s), 3. 82(3H, s), 3. 94(3H, s), 6. 82(1H, d, J=8. 5Hz), 7. 15(1H, dd, J= 8. 2, 4. 7Hz), 7. 35(1H, ddd, J=8. 2, 3. 0, 1. 4Hz), 7. 51(1H, d, J=2. 0Hz), 7. 59(1H, dd, J=8. 5, 2. 0Hz), 8. 96(1H, dd, J=4. 7, 1. 4Hz), 8. 28(1H, d, J=3Hz) | 3447 3232 3107 3030 2986 2210 1630 1581 1518 1479 1417 1321 1286 1249 1228 1211 1167 1136 1053 1035 1012 869 798 748 711 626 | mp. 113~114 ℃ white crystal | 22 |

| Example | Starting material |  | Objective material |  |  |  |  |
|---|---|---|---|---|---|---|---|
|  | Structural formula |  | Structural formula | $^1$HNMR | IR(cm-1) | mp. Form | Yield(%) |
| 25 | 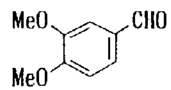 , 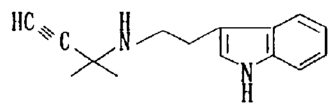 |  | 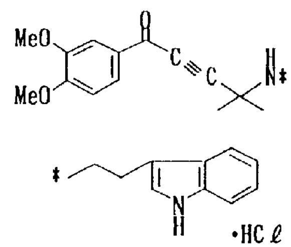 •HCℓ | (DMSO-$d_6$ 250MHz) δ ppm; 1.80(6H, s), 3.12~3.29 (2H, m), 3.30~3.42(3H, m), 3.80(3H, s), 3.88(3H, s), 6.06 ~7.00(1H, m), 7.01 ~7.09(1H, m), 7.05(1H, d, J=8.5Hz), 7.31(1H, d, J=2Hz), 7.37(1H, d, J=7.8Hz), 7.48(1H, d, J=1.8Hz), 7.60(1H, d, J=7.8Hz), 7.87 (1H, dd, J=8.5, 1.8Hz), 10.16(1H, brs), 11.00(1H, brs) | 3387 2939 2725 2449 2363 2226 1635 1581 1514 1460 1421 1346 1273 1219 1172 1143 1018 746 617 | white crystal | 74 |

| Example | Starting material | Objective material | | | | |
|---|---|---|---|---|---|---|
| | Structural formula | Structural formula | $^1$HNMR | IR(cm-1) | mp. Form | Yield(%) |
| 26 | 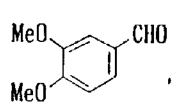 , 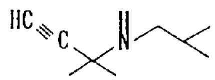 | 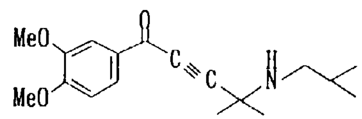 | (CDCl$_3$ 250MHz) δ ppm; 0. 96(6H, d, J=6. 6Hz), 1. 49(6H, s), 1. 80 ~1. 60(2H, m), 2. 60(2H, d, J=6. 6Hz), 3. 94(3H, s), 3. 97(3H, s), 6. 93(1H, d, J=8. 4Hz), 7. 62(1H, s), 7. 82(1H, d, J=8. 4Hz) | 3587 3323 2957 2870 2361 2339 2202 1635 1585 1514 1464 1417 1344 1271 1224 1172 1132 1024 875 819 767 750 723 652 617 | light yellowl oil | 17 |

| Example | Starting material | Objective material | | | | |
|---|---|---|---|---|---|---|
| | Structural formula | Structural formula | $^1$HNMR | IR(cm-1) | mp. Form | Yield(%) |
| 27 | 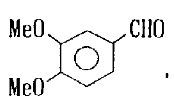 . 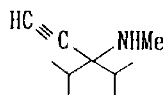 | 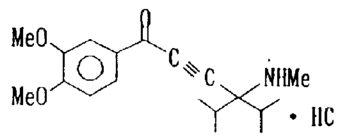 • HCℓ | ($CDCl_3$ 250MHz) δ ppm; 1.33 and 1.36(2d, J=6.7 Hz, 12H), 2.58(m, 2H), 2.98(brs, 3H), 3.95 and 3.98(2s, 6H), 6.99(d, J= 8.4Hz, 1H), 7.57(s, 1H), 7.84 (d, J=8.4Hz, 1H), 9.49(brs, 1H) | (kBr)cm-1: 3430 2963 2649 2475 2404 2216 1626 1591 1581 1516 1421 1294 1277 1175 1142 1022 | mp. 182~184 ℃ white powder | 42 |

| Example | Starting material | Objective material | | | | |
|---|---|---|---|---|---|---|
| | Structural formula | Structural formula | $^1$HNMR | IR(cm-1) | mp. Form | Yield(%) |
| 28 | 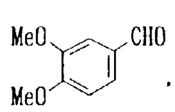 , 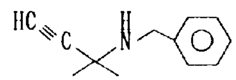 | 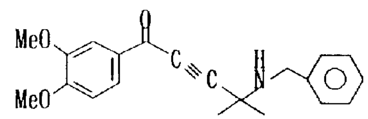 | ($CDCl_3$, 300MHz) δ ppm; 1.55(1H, brs), 1.56(6H, s), 3.94(3H, s), 3.97(5H, s), 6.93(1H, d, J=8.7Hz), 7.25 ~7.40(5H, m), 7.64(1H, d, J=1.8Hz), 7.85(1H, dd, J=8.7, 1.8Hz) | 3412 3310 3065 2984 2964 2934 2854 2700 2617 2199 1813 1753 1630 1581 1512 1454 1419 1269 1246 1174 1141 1066 1020 877 815 790 758 734 | mp. 94~ 95 ℃ white crystal | 83 |
| 29 | 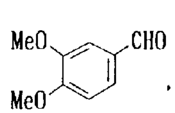 , 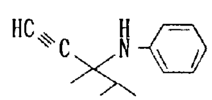 | 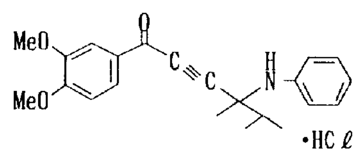 •HCℓ | ($CDCl_3$, 250MHz) δ ppm; 1.22(3H, d, J=6.7Hz), 1.28(3H, d, J=6.7Hz), 1.65 (3H, s), 2.49 ~2.56(1H, m), 3.94(3H, s), 3.09(3H, s), 6.99(1H, d, J=8.6Hz), 7.24 ~7.34(3H, m), 7.54 (1H, d, J=2Hz), 7.70(1H, dd, J=6.6, 1.2Hz), (1H, dd, J=8.6, 2.0Hz), 11.50(1H, brs) | 3412 2974 2934 2845 2623 2550 2457 2227 1890 1736 1635 1583 1568 1510 1460 1421 1271 1222 1170 1145 1114 1022 937 873 823 763 750 725 | mp. 100~103 ℃ white crystal | 39 |

| Example | Starting material | Objective material | | | | |
|---|---|---|---|---|---|---|
| | Structural formula | Structural formula | $^{1}$HNMR | IR(cm-1) | mp. Form | Yield(%) |
| 30 | 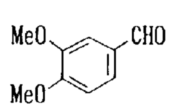 , 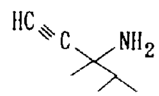 | 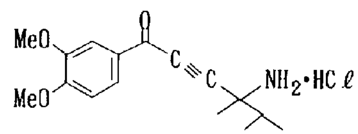 | (DMSO-$d_6$ 250MHz) δ ppm;<br>1.08(2H, d, J=6.8Hz),<br>1.12(3H, d, J=6.8Hz),<br>1.65(3H, s),<br>2.14 ~2.22(1H, m),<br>3.85 (3H, s), 3.90(3H, s),<br>7.17(1H, d, J=8.5Hz),<br>7.50(1H, s),<br>7.88(1H, d, J=8.5Hz)<br>9.02(1H, bs) | 3425 2972 2226<br>2038 1635 1583<br>1514 1466 1421<br>1348 1271 1215<br>1174 1143 1018<br>877 825 767<br>748 617 | mp. 149~151 ℃<br>white crystal | 11 |
| 31 | 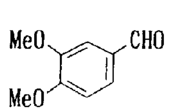 , 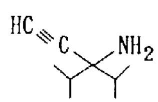 | 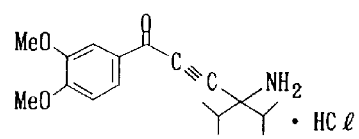 | (DMSO-$d_6$ 250MHz) δ ppm;<br>1.09(6H, d, J=6.8Hz),<br>1.15(6H, d, J=6.8Hz),<br>3.84(3H, s), 3.90(3H, s),<br>7.18(1H, d, J=8.5Hz),<br>7.50(1H, d, J=1.7Hz),<br>7.86(1H, dd, J=8.5, 1.7Hz),<br>8.75(1H, br) | 3423 2974 2878<br>2361 2341 2224<br>2035 1639 1593<br>1516 1464 1419<br>1346 1271 1211<br>1172 1141 1072<br>1020 873 848<br>823 765 748<br>721 669 650<br>617 | mp. 94~ 97 ℃<br>white crystal | 4 |

| Example | Starting material | Objective material | | | | |
|---|---|---|---|---|---|---|
| | Structural formula | Structural formula | $^1$HNMR | IR(cm-1) | mp. Form | Yield(%) |
| 32 | 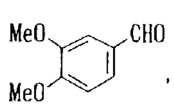 , 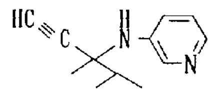 | 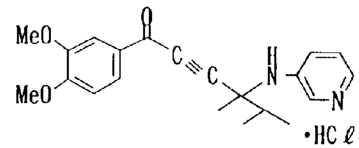 | (CDCl$_3$, 350MHz) δ ppm; 1.17(3H, d, J=6.6Hz), 1.33 (3H, d, J=6.6Hz), 1.72(3H, s), 2.41 ~2.51(1H, m), 3.91(3H, s), 3.96(3H, s), 6.89(1H, d, J =7.5Hz), 7.51 ~7.55(1H, m), 7.56(1H, d, J=1.8Hz), 7.70(1 H, dd, J=8.3, 2.0Hz), 7.76(1 H, brs), 8.03(1H, dd, J=7.5, 1.8Hz), 9.20~9.30(1H, m), 11.38(1H, brs) | 3412 3246 2970 2970 2361 1556 1514 1466 1419 1346 1273 1222 1172 1132 1018 873 794 748 721 679 617 | mp. 103 ~ 106 ℃ light yellowl crystal | 26 |
| 33 | 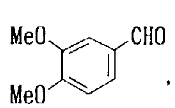 , 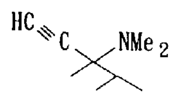 | 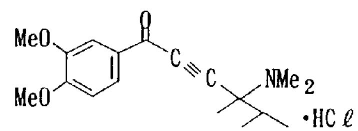 | (CDCl$_3$, 300MHz) δ ppm; 1.35(3H, d, J=6.6Hz), 1.36 (3H, d, J=6.6Hz), 1.78(3H, s), 2.41(1H, m), 2.95(3H, d, J=4.2Hz), 3.00(3H, d, J=4.2Hz), 3.95(3H, s), 3.99 (3H, s), 7.00(1H, d, J=8.4, 8.4Hz), 7.55(1H, d, J=1.8 Hz), 7.83 (1H, dd, J=8.4, 1.8Hz), 12.48 (1H, brs) | 3531 2972 2843 2507 2409 2224 1724 1630 1581 1516 1462 1425 1234 1213 1178 1145 1126 1091 1020 918 875 848 810 769 746 719 | mp. 178 ~ 180 ℃ white crystal | 42 |

| Example | Starting material: Structural formula | Objective material: Structural formula | Objective material: $^1$HNMR | Objective material: IR(cm-1) | Objective material: mp. Form | Objective material: Yield(%) |
|---|---|---|---|---|---|---|
| 34 | 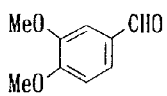 , 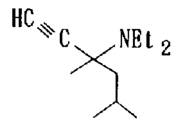 | 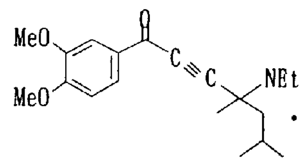 • HCℓ | ($CDCl_3$, 300MHz) δ ppm; 1.10(3H, d, J=6.3Hz), 1.15(3H, d, J=6.3Hz), 1.61(6H, t, J=7.5Hz), 2.02(3H, s), 2.03 ~2.09(2H, m), 2.36 ~2.49(1H, m), 3.20~3.35(2H, m), 3.47~3.64(2H, m), 3.95(3H, s), 3.98(3H, s), 6.98(1H, d, J=8.9Hz), 7.54(1H, d, J=2.1Hz), 7.83(1H, dd, J=8.4, 2.1Hz), 12.30(1H, brs) | 3420 3078 2939 2534 2336 2218 1628 1581 1520 1456 1439 1421 1282 1248 1172 1153 1089 1020 891 864 769 744 723 659 | mp. 145~147 ℃ white crystal | 89 |
| 35 | 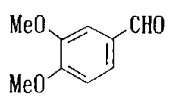 , 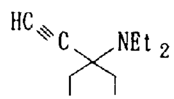 | 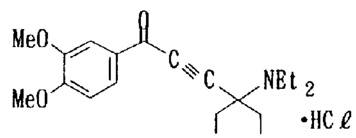 •HCℓ | ($CDCl_3$, 300MHz) δ ppm; 1.28(6H, d, J=7.5Hz), 1.60(6H, t, J=7.5Hz), 2.20~2.49(2H, m), 2.35~2.50(2H, m), 3.22~3.40(2H, m), 3.50~3.67(2H, m), 3.95(3H, s), 3.98(3H, s), 6.98(1H, d, J=8.1Hz), 7.55(1H, d, J=1.8Hz), 7.80(1H, dd, J=8.1, 1.8Hz), 12.10(1H, brs) | 3427 2978 2604 2417 2363 2214 1631 1579 1514 1454 1421 1288 1273 1249 1222 1168 1141 1016 910 871 814 790 765 746 719 628 513 432 | mp. 139.5~14.05 ℃ white crystal | 45 |

| Example | Starting material | Objective material | | | | |
|---|---|---|---|---|---|---|
| | Structural formula | Structural formula | $^1$HNMR | IR(cm-1) | mp. Form | Yield(%) |
| 36 | 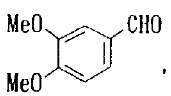 , 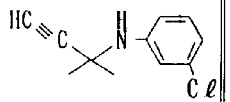 | 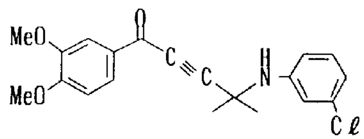 ·HCℓ | (DMSO-$d_6$ 250MHz) δ ppm; 1.69(6H, s), 3.73(3H, s), 3.86(3H, s), 5.92(2H, br), 6.70 ~6.78(1H, m), 6.93(1H, d, J=8.0Hz), 6.99(1H, s), 7.08(1H, d, J=8.4Hz), 7.19(1H, dd, J=8.0, 8.0Hz), 7.43(1H, d, J=1.8Hz), 7.67(1H, dd, J=8.4, 1.8Hz) | | light yellow crystal | 27 |

Example 37

Synthesis of hydrochloride of compound No. 57 in Table-1

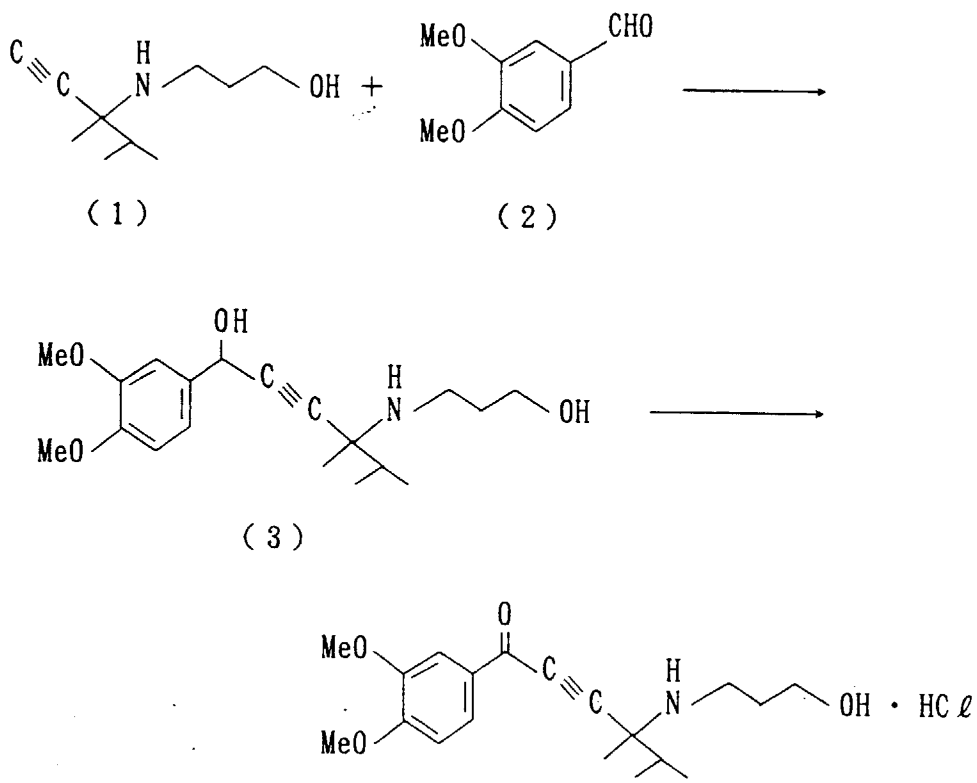

(1) (2) (3) (4)

Under a nitrogen atmosphere 2.8ml of n-butyllithium (1.6M in hexane) was added dropwise to a solution of the above-noted amine (1) (0.38g, 2.25mmol) in anhydrous tetrahydrofuran at -78°C (20ml) and the resulting solution was stirred for 1 hour. To this solution was added a solution of veratoraldehyde (2) (0.37g, 2.25mmol) in anhydrous tetrahydrofuran (5ml) and the solution was stirred for 1 hour. Water was added to the solution and the solvent was distilled off under reduced pressure. After the residue was extracted with 2N HCl aqueous solution and the aqueous layer was washed with ethyl acetate, 2N NaOH a solution was added to the aqueous layer to make pH above 13, and the mixture extracted with dichloromethane. After drying organic layer over sodium sulfate, the solvent was distilled off under reduced presure. The residue obtained was washed by a silica gel column chromatography (ethyl acetate→ chloroform:methanol=8:1) to obtain 0.46g of alcohol compound (3) (yield 60%).

Manganese dioxide (200mg) was added to a solution of the alcohol compound (3) (36mg, 0.10mmol) in anhydrous dichloromethane (5ml) and stirred at the room temperature. After insolubles were filtered off and 25µl of 4N HCl-ethyl acetate solution was added to a filtrate, the solvent was distilled off to obtain 10mg (yield 27%) of (4) as hygroscopic crystals.

$^1$H NMR ($CDCl_3$, 250 MHz) δ ppm :

1.21 (3H, d, J=6.6Hz),
1.27 (3H, d, J=6.6 Hz),
1.79 (3H, s),
1.96~2.13 (2H, m),
2.27~2.45 (1H, m),
2.50~2.69 (1H, m),
3.42~3.65 (2H, m),
3.83~3.98 (1H, m),

3.95 (3H, s),
3.98 (3H, s),
4.05~4.29 (1H, m),
7.05 (1H, d, J=8.5 Hz),
7.57 (1H, d, J=1.9 Hz),
7.95 (1H, dd, J=8.5, 1.9 Hz),
9.24 (1H, brs),
10.60 (1H, brs).

Example 38

Synthesis of hydrochloride of compound No. 58 in Table-1

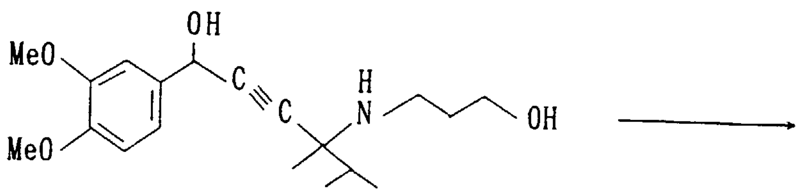

( 3 )

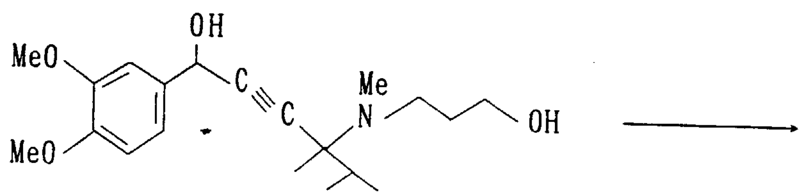

( 5 )

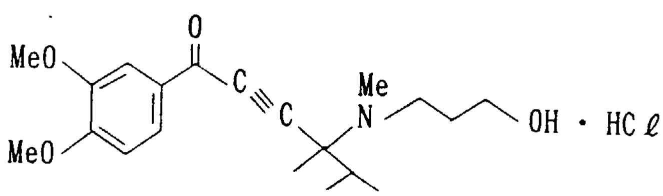

( 6 )

35% aqueous formaldehyde solution was added to a solution of the alcohol compound (3) (150mg, 0.45mmol) synthesized in Example 61 in acetonitrile (0.2ml, 2.3mmol), then 71mg (0.90mmol) of $NaBH_3CN$ was added and stirred for 2 hours at room temperature. After 3ml of glacial acetic acid was added dropwise, the reaction mixture was concentrated under reduced pressure, to which 4N KOH aqueous solution was added to make basic, and then extracted with ether. After the organic layer was extracted with 1N HCl aqueous solution, an aqueous layer was made basic with 4N KOH aqueous solution, and extracted with ether. After the organic layer was dried over anhydrous magnesium sulfate, the solvent was distilled off under reduced pressure, and the residue was purified by a silica gel column chromatography (chloroform:methanol=20:1) to give 98mg (yield 62%) of (5).

350mg of manganese dioxide gas was added to a solution of (5) (98mg) in dichloromethane (5ml) and stirred for 1 hour at room temperature, and thereafter insolubles were filtered off and 0.12ml of 4N HCl-ethyl acetate solution to a residue, then the solvent was distilled off to obtain 45mg (yield 41%) of (6).

$^1H$ NMR ($CDCl_3$, 300 MHz) $\delta$ ppm :

1.35 (6H, d, J=6.6 Hz),
1.83 (3H, s),
1.93~2.20 (4H, m),
2.40~2.60 (2H, m),
3.07 (3H, s),
3.1 2~3.31 (1H, m),
3.67~3.83 (2H, m),
3.89 (3H, s),
3.99 (3H, s),
7.00 (1H, d, J=8.4 Hz),
7.54 (1H, d, J=2.1 Hz),
7.88 (1H, dd, J=8.4, 2.1 Hz),
11.40 (1H, brs).

Test Example 1

<Evaluation of tyrosine kinase inhibitor of the present invention>

In order to evaluate tyrosine kinase inhibitory activity and cancer cell growth inhibitory activity for tyrosine kinase inhibitor of the invention, tests were carried out in partially purified human EGF (epidermal growth factor) receptor tyrosine kinase activity detemination system and in a cell incubation system using human cancer cells. Furthermore, in order to compare and evaluate strength of inhibitory activity, relatively higher active ones amongst known tyrosine kinase inhibitors disclosed in patents or literatures were tested simultaneously.

(1) Tyrosine kinase inhibitory activity

(Determination method)

Tyrosine kinase activity was measured using partially purified EGF receptor prepared from A431 cell line derived from human epidermoid carcinoma according to the improved method of that of Linda J. Pike et al (Proceedings of the National Academy of Sciences of the U.S.A., 79 1443, 1982).

The method employed is detailed below.

A431 cells were cultured in Dulbecco's modified Eagles medium (DMEM) containing 10% fetal calf serum (FCS) at 37°C under 5% $CO_2$ atmosphere. The cultured cells were homogenated in a solution containing 10mM N-2-hydroxyethylpiperazino-N'-2-ethane sulfonate buffer (pH 7.4), 0.25M sucrose, and 0.1mM EDTA, and centrifuged at 3000×g for 5 minutes. The supernatant was then centrifuged at 10000×g for 30 minutes to obtain A431 cell membrane fraction. The cell membrane fraction, a partially purified EGF receptor, was used as an enzyme source. A test compound dissolved in dimethylsulfoxide (DMSO) was added to a reaction mixture containing 15mM Hepes buffer (pH 7.7), 2mM $MnCl_2$, 10μM $ZnSO_4$, 50μM $Na_3VO_4$ and the partially purified EGF receptor preparation (10-15 μg) (final concentration 1% DMSO). To this mixture 100ng EGF, 75μg synthetic substrate RR-SRC peptide shown by Sequence No. 1 in Sequence table and 10μM γ-$^{32}$P-adenosine triphosphate (55.5 KBq) were added thereto to start the reaction. The volume at that time was 60 μl. RR-SRC peptide is a substrate for tyrosine kinase of EGF receptor and has an amino acid sequence containing tyrosine residue which is phosphorated in a gene product of src. The mixture was allowed to react under ice-cold for 30 minutes, and the reaction was stopped by addition of 10 mg/ml bovine serum albumin and 20% trichloroacetic acid.

The reaction mixture was left to stand under ice-cold for 30 minutes. The mixture was centrifuged at 5000×g for two minutes, and a 40 μl aliquot of the supernatant was adsorbed on P81 phosphocellulose paper. The paper was dipped in 30% aqueous acetic acid for 15 minutes for fixation and then washed with 15% acetic acid. The washing was repeated four times. The radioactivity of $^{32}$P retained on the phosphocellulose paper was counted with liquid scintillation counter. The value is A.

Count numbers in the reaction without addition of test material as well as in the reaction without addition of test material and EGF were determined simultaneously, those values being B and C respectively.

Tyrosine kinase inhibitory ratio can be calculated from those values according to the following formula:

$$\text{Inhibitory rate (\%)} = \{(B-A)/(B-C)\} \times 100$$

The value $IC_{50}$ value (concentration of a test compound providing 50% inhibition) was calculated from inhibitory rate at various concentrations of a test compound in the above-described test procedure.

Cancer Cell Growth Inhibitory Activity

KB cell, which is a human rhinopharyngeal cancer cell, possesses overexpression of EGF receptors. Accordingly, the KB cell was used to evaluate the effect of test compounds on the growth of cultured cancer cells.

KB cells were plated on a 96-well dish ($2.5\times10^3$ cell/well) and cultured for 24 hours at 37°C under 5% $CO_2$ atmosphere in DMEM:F12 (1:1) medium containing 10% FCS, 50 μg/ml penicillin, and 50μg/ml streptomycin. To the culture medium was added a test compound dissolved in DMSO (DMSO final concentration < 0.1%), and the cells were cultured for three days under the same conditions. The test compound was exchanged every 24 hours together with the culture medium.

The amount of living cells were measured by calorimetric determination at wave lengths of 550 nm and 650 nm after developing colour by MTT (3-[4,5-Dimethylthiazol-2-yl]-2,5-diphenyltetrazolium bromide), in accordance with the method of Michael C. Alley et al. (Cancer Research 48 589, 1988).

Cancer cell growth inhibitory activity (CCGIA) was determined according to the following equation.

$$\mathrm{CCGIA}\ (\%) = (b-a)/b \times 100$$

Symbols "a" and "b" represent the colorimetric values determined in the cancer cell growth assay with and without the test compounds, respectively. The value $IC_{50}$ (concentration of a test compound providing 50% inhibition) was calculated from CCGIA values at various concentration of a test compound in the above-described procedure.

Results are shown in Table-2 and Table-3, wherein "Compound No." in the first column corresponds to that in Table-1.

1) A.Gazit, N.Osheroy, I.Posner, P.Yaish, E.Pradosu, C.Gilon and A.Levitzki; J. Med. Chem., 34, 1896(1991).
2) T.Shiraishi, K.Kameyama, N.Imai, T.Domoto, I.Katsumi and K.Watanabe; Chem. Pharm. Bull., 36, (1988).
3) fumarate, 4) hydrochloride

From the above-noted results, it is clear that the compounds according to the invention are superior in tyrosine kinase inhibiting activity and cancer cell growth inhibiting activity.

[Sequence Table]

Sequence number: 1

Length of sequence: 13

Type of sequence: amino acid

Topology: straight chain

Kind of sequence: peptide

Sequence

```
Arg Arg Leu Ile Glu Asp Ala Glu Tyr Ala Ala Arg Gly
 1               5                  10
```

Table - 2

| Compound No. (Example No.) | Tyrosine kinase inhibitory activity (IC$_{50}$ , $\mu$M) |
|---|---|
| 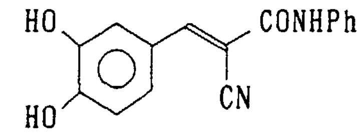 1 ) | 3. 9 |
| 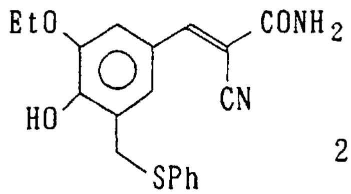 2 ) | 3. 2 |
| 6 (Example 1) | 0. 0 3 5 |
| 9 (Example 2) 3) | 0. 4 7 |
| 10 (Example 3) | 0. 1 8 |
| 24 (Example 4) | 1. 3 |
| 17 (Example 5) 4) | 0. 7 3 |
| 30 (Example 6) | 1. 7 |
| 22 (Example 7) | 2. 9 |
| 1 (Example 9) | 0. 2 4 |
| 15 (Example 10) | 0. 0 3 9 |
| 41 (Example 11) | 0. 6 6 |
| 53 (Example 14) | 0. 9 6 |
| 55 (Example 15) | 0. 6 6 |

Table — 2 (continued)

| Compound No. (Example No.) | Tyrosine kinase inhibitory activity ($IC_{50}$ . μ) |
|---|---|
| 54 (Example 16) | 0. 4 3 |
| 14 (Example 17) | 0. 0 6 9 |
| 5 (Example 20) 4) | 0. 0 1 3 |
| 16 (Example 21) 4) | 0. 3 3 |
| 47 (Example 22) 4) | 0. 1 2 |
| 49 (Example 23) | 2. 2 |
| 50 (Example 24) | 0. 3 |
| 52 (Example 25) 4) | 0. 0 3 7 |
| 48 (Example 26) | 0. 0 6 6 |
| 51 (Example 28) | 0. 1 9 |
| 62 (Example 31) 4) | 1. 1 |
| 68 (Example 34) 4) | 1. 3 |
| 18 (Example 35) 4) | 2. 2 |
| 46 (Example 36) | 0. 0 9 6 |
| 57 (Example 37) 4) | 0. 9 4 |

Table - 3

| Compound No. (Example No.) | Cancer cell growth inhibitory activity($IC_{50}$, $\mu M$) |
|---|---|
| 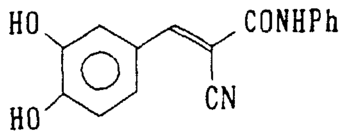 1) | 17 |
| 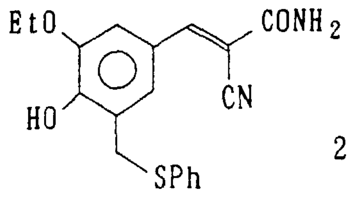 2) | 52 |
| 6 (Example 1) | 2.1 |
| 9 (Example 2) 3) | 1.6 |
| 10 (Example 3) | 1.0 |
| 24 (Example 4) | 0.64 |
| 17 (Example 5) 4) | 0.69 |
| 30 (Example 6) | 0.46 |
| 22 (Example 7) | 0.56 |
| 1 (Example 9) | 1.52 |
| 41 (Example 11) | 1.1 |

Table − 3 (continued)

| Compound No. (Example No.) | Cancer cell growth inhibitory activity($IC_{50}$, $\mu M$) |
|---|---|
| 53 (Example 14) | 1.1 |
| 55 (Example 15) | 3.5 |
| 54 (Example 16) | 6.5 |
| 14 (Example 17) | 1.1 |
| 56 (Example 19) | 1.3 |
| 5 (Example 20) 4) | 0.84 |
| 16 (Example 21) 4) | 0.93 |
| 47 (Example 22) 4) | 1.1 |
| 49 (Example 23) | 3.4 |
| 50 (Example 24) | 2.8 |
| 52 (Example 25) 4) | 1.6 |
| 48 (Example 26) | 2.8 |
| 63 (Example 27) 4) | 2.4 |

Table –

| Compound No. (Example No.) | Cancer cell growth inhibitory activity($IC_{50}$ , μM) |
|---|---|
| 51(Example 28) | 0. 7 9 |
| 61(Example 29) 4) | 0. 4 8 |
| 62(Example 31) 4) | 9. 7 |
| 60(Example 33) 4) | 2. 0 |
| 668(Example 57) 4) | 0. 0 7 5 |
| 18(Example 35) 4) | 0. 2 2 |
| 46(Example 36) | 1. 5 |
| 667(Example 60) | 0. 1 2 |
| 57(Example 37) 4) | 1. 0 |
| 58(Example 38) 4) | 2. 1 |
| 677(Example 64) 4) | 1. 2 |

## Claims

1. A compound of the following formula (I)

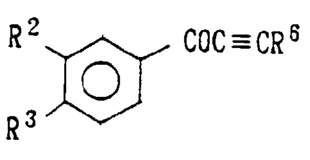

··· ( I )

wherein $R^2$ and $R^3$ are $C_1$-$C_3$ alkoxyl $R^6$ is phenyl, pyridyl or -$CR^{15}R^{16}X$ wherein $R^{15}$ and $R^{16}$ are independently hydrogen or $C_1$-$C_5$ alkyl. optionally substituted by phenyl, or when taken together, they are $C_3$-$C_7$ alkylene, X is hydroxyl or -$NR^{23}R^{24}$ wherein $R^{23}$ and $R^{24}$ are independently hydrogen, phenyl optionally substituted by halogen or $C_1$-$C_5$ alkyl, $C_1$-$C_5$ alkyl optionally substituted by hydroxyl, phenyl or indolyl, pyridyl, -$SO_2R^{27}$ wherein $R^{27}$ is 3,4-dimethoxyphenyl, or -$COR^{25}$ wherein $R^{25}$ is $C_1$-$C_5$ alkoxy optionally substituted by phenyl, pyridyl, N-methylpydridyl or N-oxopyridyl, or when $R^{23}$ and $R^{24}$ are taken together, they are $C_3$-$C_6$ alkylene optionally substituted by $C_1$-$C_5$ alkyl; or its salt, except 2-propyn-1-one, 1-(3,4-dimethoxyphenyl)-3-phenyl.

2. A tyrosine kinase inhibitor. which comprises as an essential ingredient a benzoylacetylene derivative of formula (I) as defined in claim 1 or its salt.

3. A pharmaceutical composition which comprises a compound of claim 1 and pharmaceutically acceptable carriers.

4. The composition of claim 3 for suppressing the growth of cancer cells.

5. The composition of claim 4, wherein the cancer cell is of stomach cancer, breast cancer, ovary cancer, lung cancer, colon cancer. brain cancer, leukemia, pancreatic cancer or esophageal cancer.

6. The composition of claim 3 for therapeutical treatment of arterial sclerosis.

7. The composition of claim 3 for inhibiting platelet aggregation.

8. The composition of claim 3 for immunodepression.

9. The composition of claim 3 for suppressing inflammation.

**Patentansprüche**

1. Verbindung der folgenden Formel (I)

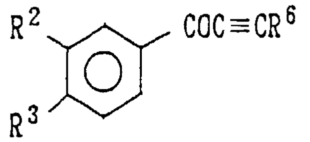

... ( I )

worin $R^2$ und $R^3$ $C_1$-$C_3$ Alkoxy sind; $R^6$ Phenyl, Pyridyl oder -$CR^{15}R^{16}X$ ist, worin $R^{15}$ und $R^{16}$ unabhängig voneinander Wasserstoff oder $C_1$-$C_5$-Alkyl, wahlweise substituiert durch Phenyl, oder wenn zusammengenommen, $C_3$-$C_7$-Alkylen sind, X Hydroxyl oder -$NR^{23}R^{24}$, worin $R^{23}$ und $R^{24}$ unabhängig voneinander Wasserstoff, Phenyl, wahlweise substituiert durch Halogen oder $C_1$-$C_5$-Alkyl, $C_1$-$C_5$-Alkyl, wahlweise substituiert durch Hydroxyl, Phenyl oder Indolyl, Pyridyl, -$SO_2R^{27}$, worin $R^{27}$ 3,4-Dimethoxyphenyl ist, oder -$COR^{25}$, worin $R^{25}$ $C_1$-$C_5$-Alkoxy ist, wahlweise substituiert durch Phenyl, Pyridyl, N-Methylpyridyl oder N-Oxopyridyl, oder wenn $R^{23}$ und $R^{24}$ zusammengenommen werden, sie $C_3$-$C_6$-Alkylen, wahlweise substituiert durch $C_1$-$C_5$-Alkyl, sind; oder deren Salz, mit Ausnahme von 2-Propin-1-one, 1-(3,4-dimethoxyphenyl)-3-phenyl.

2. Tyrosinkinaseinhibitor, umfassen als wesentlichen Bestandteil ein Benzoylacetylenderivat der Formel (I), wie in Anspruch 1 definiert, oder dessen Salz.

3. Pharmazeutische Zusammensetzung, umfassend eine Verbindung nach Anspruch 1 und pharmazeutisch annehmbare Träger.

4. Zusammensetzung nach Anspruch 3 zur Unterdrückung des Wachstums von Krebszellen.

5. Zusammensetzung nach Anspruch 4, wobei die Krebszelle von einem Magenkrebs, Brustkrebs, Eierstockkrebs, Lungenkrebs, Kolonkrebs, Hirnkrebs, Leukämie, Bauchspeicheldrüsenkrebs oder Speiseröhrenkrebs stammt.

6. Zusammensetzung nach Anspruch 3 zur therapeutischen Behandlung von Arteriosklerose.

7. Zusammensetzung nach Anspruch 3 zur Inhibierung der Blutplättchenaggregation.

8. Zusammensetzung nach Anspruch 3 für die Immunodepression.

9. Zusammensetzung nach Anspruch 3 zur Unterdrückung einer Entzündung.

**Revendications**

1. Composé de formule (I) suivante

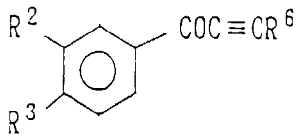

··· ( I )

dans laquelle $R^2$ et $R^3$ sont des groupes alcoxy en $C_1$-$C_3$, $R^6$ est un groupe phényle, pyridyle ou $-CR^{15}R^{16}X$ dans lequel $R^{15}$ et $R^{16}$ sont indépendamment un atome d'hydrogène ou un groupe alkyle en $C_1$-$C_5$ éventuellement substitué par un groupe phényle, ou lorsqu'ils sont pris ensemble ils représentent un groupe alkylène en $C_3$-$C_7$, X est un groupe hydroxyle ou $-NR^{23}R^{24}$ dans lequel $R^{23}$ et $R^{24}$ sont indépendamment un atome d'hydrogène, un groupe phényle éventuellement substitué par un atome d'halogène ou par un groupe alkyle en $C_1$-$C_5$, un groupe alkyle en $C_1$-$C_5$ éventuellement substitué par un groupe hydroxyle, phényle ou indolyle, pyridyle, $-SO_2R^{27}$ dans lequel $R^{27}$ est un groupe 3,4-diméthoxyphényle ou $-COR^{25}$ dans lequel $R^{25}$ est un groupe alcoxy en $C_1$-$C_5$ éventuellement substitué par un groupe phényle, pyridyle, N-méthylpyridyle ou N-oxo-pyridyle, ou lorsque $R^{23}$ et $R^{24}$ sont pris ensemble, ils représentent un groupe alkylène en $C_3$-$C_6$ éventuellement substitué par un groupe alkyle en $C_1$-$C_5$; ou un de ses sels, à l'exception de la 2-propyn-1-one et du 1-(3,4-diméthoxyphényl)-3-phényle.

**2.** Inhibiteur de la tyrosine-kinase comprenant en tant qu'ingrédient essentiel un dérivé du benzoylacétylène de formule (I) tel que défini dans la revendication 1 ou un de ses sels.

**3.** Composition pharmaceutique comprenant un composé selon la revendication 1 et des véhicules pharmaceutiquement acceptables.

**4.** Composition selon la revendication 3 servant à empêcher la croissance des cellules cancéreuses.

**5.** Composition selon la revendication 4, dans laquelle la cellule cancéreuse est une cellule de cancer de l'estomac, de cancer du sein, de cancer des ovaires, de cancer des poumons, de cancer du côlon, de cancer du cerveau, de leucémie, de cancer pancréatique ou de cancer oesophagien.

**6.** Composition selon la revendication 3 utilisée pour le traitement thérapeutique de l'artériosclérose.

**7.** Composition selon la revendication 3 servant à inhiber l'agrégation plaquettaire.

**8.** Composition selon la revendication 3 utilisée pour l'immuno-dépression.

**9.** Composition selon la revendication 3 utilisée pour supprimer l'inflammation.